# EUROPEAN PATENT SPECIFICATION

(11) **EP 2 746 405 B1**
(45) Date of publication and mention of the grant of the patent: **04.11.2015**
(21) Application number: 13175199.2
(22) Date of filing: 04.07.2013
(51) Int. Cl.: C12Q 1/68

(54) **Methods and primer sets for high throughput PCR sequencing**
Verfahren und Primersätze zur PCR-Hochdurchsatzsequenzierung
Procédés et ensembles d'amorces de séquençage de PCR à haut rendement

(30) Priority: 23.12.2012 EP 12199315
(43) Date of publication of application: 25.06.2014
(73) Proprietor: HS Diagnomics GmbH, 10961 Berlin (DE)
(72) Inventor: Hennig, Steffen, 10961 Berlin (DE); Seitz, Volkhard, 12167 Berlin (DE); Ritter, Julia-Marie, 12157 Berlin (DE); Hummel, Michael, 12105 Berlin (DE)
(74) Representative: Schulz Junghans Patentanwälte PartGmbB

(56) References cited:
- WO-A1-91/15601
- WO-A1-99/20798
- MACHTELD BAETENS ET AL: "Applying massive parallel sequencing to molecular diagnosis of Marfan and Loeys-Dietz syndromes", HUMAN MUTATION, vol. 32, no. 9, 20 September 2011 (2011-09-20), pages 1053-1062, XP055075059, ISSN: 1059-7794, DOI: 10.1002/humu.21525
- ADAM LEVY/ AMPLION LTD: "Two-temperature tagged (2T-TA) PCR for elimination of false positives due to amplicon contamination", RESEARCH DISCLOSURE, MASON PUBLICATIONS, HAMPSHIRE, GB, vol. 499, no. 7, 14 October 2005 (2005-10-14), XP007135635, ISSN: 0374-4353

## Description

Innovative techniques have been recently developed that allow the parallel generation of millions of sequence reads in a single run. High-dimensional data derived from this "next generation" or high throughput sequencing (NGS or HTS) may be used to resolve the biological variability within a single individual or within a population to a hitherto unknown precision and depth. Due to the extremely high sensitivity of these advanced sequencing technologies, however, (cross-)contamination become detectable which previously escaped detection by application of less sensitive methods.

Contamination was also underestimated when PCR was introduced and widely used in the in the eighties and early nineties. Therefore, a substantial of initial data appears to contain artificial results. Contamination is even at higher risk for NGS/HTS techniques, where high sensitive PCR techniques combined with massive parallel sequencing are able to display each individual sequence. In order to avoid misinterpretation of NGS/HTS data, it is mandatory to introduce methods that
(i) prevent the production of contaminating sequences or
(ii) identify the presence of contaminating sequences.

The first option is of highest priority which is in cooperation with identification of potential contaminations decisive for accurate medical diagnostics.

There are several sources for contamination in the course of amplicon sequencing. One major source is associated with the fact that two subsequent PCRs (so-called nested PCR) are required prior to sequencing. In the first amplification reaction, the target nucleic acid is amplified with primers specific for a given usually genomic target. In a second amplification reaction, nucleic acid sequences required for sequencing are added to the product of the first amplification reaction. Due to the high number of amplicons generated in the first amplification reaction, there is a high risk for cross-contamination by carry-over of amplicons from the first PCR to the re-amplification. In the case of amplicon isolation by gel extraction or PCR-purification kits, the risk of contamination is even higher.

Baetens et al. (Human Mutation Vol. 32, 2053-62 (2011)) show a two-step amplification-sequencing process using 5'-tagged first primers to yield a first amplificate, which are amplified in a second step to yield the sequenced amplificate.

Levy (Research disclosure database 499007, Kenneth Mason Publications, 2005) shows a two-step, two temperature PCR amplification process by which amplion contamination in the sample is detected by occurrence of amplificates at a higher annealing temperature.

WO99/20798 describes a PCR amplification process using different primers specific for sample and primer to detect amplicon contamination from previous amplification reactions.

The objective of the present invention is to (i) avoid and/or to (ii) detect PCR-based contamination in applications employing massive parallel sequencing (NGS/HTS) techniques. The objective of this invention is attained by the subject matter of the independent claims.

### Terms and definitions

Nucleic acid sequences are given from 5' to 3' end. A sequence tract in the context used herein refers to a contiguous sequence; a sequence tract designator is a letter, optionally having a subscript or superscript, representing a sequence tract. k₁, k₁', k₂, k₂', s and s' are examples for sequence tract designators. Where sequences are given as a sequence of sequence tract designators, such sequences are understood to be ordered similarly in canonical 5' to 3' order. A sequence tract is also called a sequence element.

Nucleic acid target sequences may be DNA or RNA; in case of RNA as a target sequence for amplification and sequencing, RNA is transcribed into cDNA (by reverse transcriptase) prior to amplification.

A "primer" in the context of the present specification refers to a single stranded DNA oligomer having a length between 8 and 100 nucleotides.

"Capable of forming a hybrid" in the context of the present invention relates to sequences, which are able to bind selectively to their target sequence under the conditions of a given PCR or sequencing reaction (for example, 10 mmol/l Tris-HCl pH 8.3; 100 mmol/l KCI; 1.5 mmol/l MgCl₂; 0.2 mmol/l dNTP, each; primer annealing temperature of 40°C to 68°C). Such hybridizing sequences may be contiguously reverse-complimentary to the target sequence, or may comprise gaps, mismatches or additional non-matching nucleotides. The minimal length for a sequence to be capable of forming a hybrid depends on its composition, with C or G nucleotides contributing more to the energy of binding than A or T/U nucleotides, and on the backbone chemistry.

"Nucleotide" in the context of the present invention is a nucleic acid or nucleic acid analogue building block, an oligomer of which is capable of forming selective hybrids with an RNA or DNA sequence on the basis of base pairing. The term *nucleotides* in this context includes the classic ribonucleotide building blocks adenosine, guanosine, uridine (and ribosylthymine), cytidine, and the classic deoxyribonucleotides deoxyadenosine, deoxyguanosine, thymidine, deoxyuridine and deoxycytidine. The term *nucleotides* further includes analogues of nucleic acids such as phosphorotioates, 2'O-methylphosphothioates, peptide nucleic acids (PNA; N-(2-aminoethyl)-glycine units linked by peptide linkage, with the nucleobase attached to the alpha-carbon of the glycine) or *locked nucleic acids* (LNA; 2'O, 4'C methylene bridged RNA building blocks). A *primer sequence* as used in the context of the present specification may be composed of any of the above nucleotides, or mixtures thereof. In some embodiments, a primer sequence is composed of deoxynucleotides, with the last (from the 3' position) 1, 2, 3 or 4 internucleotide bonds being phosporothioates. In some embodiments of the invention the last 1-4 nucleotides (from the 3'position) are LNAs.

### Summary of the invention

The present invention provides guidelines for the design of three synergistically acting primer elements (designated generally as K-box with a capital "K" being subdivided in a k-box (written with a lower case "k") for the forward primers and a k'-box for the reverse primers respectively) which in combination greatly improve accuracy of PCR library preparations that can be analysed by massively parallel ("high throughput") sequencing reactions.

The method of the invention makes use of two primer pairs. The first or initial primer pair amplifies the target sequence generating a first amplificate. Subsequently, a nested second (adaptor) primer pair amplifies the first amplificate producing the second amplificate, which can be used for sequencing. The first and second primer pairs are individualized as "sets" whereby a specific second primer pair is designed to only work together with a specific first primer pair within a set.

The first and second primer pairs of a set comprise a sequence tract referred to as K-box. Each K-box is specific for a set. The K-box of the (initial) primers for the first amplification step can comprise different elements k₁, k₁', k₂, k₂', S and S'. As explained in detail below, S/S' prevent a PCR bias, k₂/k₂' serve to detect contaminations and k₁/k₁', which are also present in the K-box of the second amplification primers prevent contamination. Importantly, matching k₁/k₁' sequences enable the second primer pair to amplify the first amplificate only if the matching k₁/k₁' sequences were comprised in the first primer of the same set that was used to generate the first amplificate. In other words, if there is no k₁/k₁' matching, the second primer pair does not amplify the first amplificate, thereby resulting in contamination prevention. Thus, primer pairs are arranged in corresponding and matching sets. A number of sets (e.g. set 1 - 300) are a collection.

All initial primers of the first amplification step of a collection amplify the same target sequence. Different collections, amplifying different target sequences (i.e. in a multiplex PCR), may be combined as a library. A library can be also understood as a multiplex-collection.

The combination of all three K-box elements provides a most efficient protection of contamination, whereas the individual elements alone (k₁/k₁', k₂/k₂', S/S') cover only single aspects of contamination protection/detection. E.g. k₁/k₁' and k₂/k₂' depend on s/s' to avoid a PCR bias, as outlined below. There is also an interdependence between the K-box and the other primer sequences. Therefore, the three K-box elements are bioinformatically designed as one specific K-box for each primer set and optimized not to form hybrids with the 3'ends of the primers employed. As a result there is only a limited number of K-boxes suitable for contamination protection in a certain application depending e.g. on S/S' sequence composition and the length of k₁/k₁', k₂/k₂'.

### Detailed description of the invention

### Overview of the target and primer sequence tracts

The target nucleic acid sequences are described as t_{C}-t_{V}-t_{C}'. Thereby t_{C}/t_{C}' are the primer-hybridizing sequence tract for the forward (left) and reverse (right) primary amplification primer respectively. t_{V} is a variable region within a target nucleic acid sequence. Furthermore the target nucleic acid sequence sequence elements tₙ and tₙ' are located in 5' and 3' position, respectively. In other words, the target structure can be described as tₙ-t_{C}-t_{V}-t_{C}'-tₙ'.

A primer for use in a method or collection of primers according to the invention is composed of at least two sequence tracts.

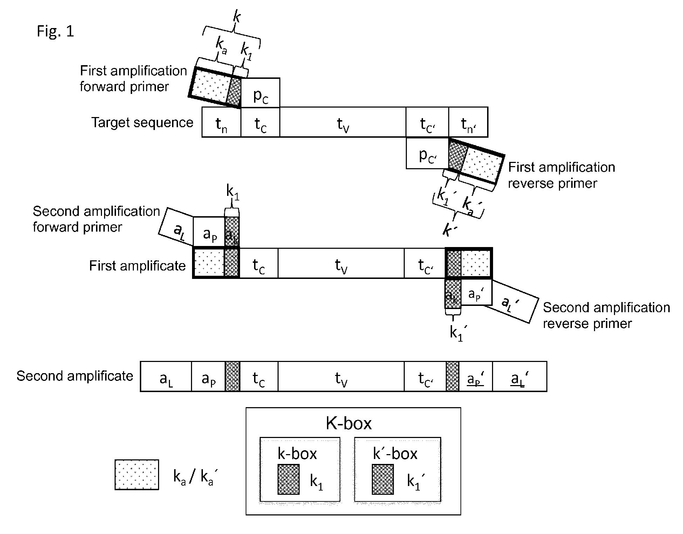

A first or initial primer used in the first round of amplification comprises, in order from 5' to 3' OH-end, a sequence tract kₐ-k-box (also designated as k) and a sequence tract p_{C} (Fig. 1). Sequence tract p_{C} provides target specificity, while k provides a non-target-specific sequence tract, parts or all of which can be used for hybridization of a second "adaptor" primer. Within k the sequence tract kₐ can comprise sequence elements necessary for sequencing purposes and the K-box comprises the K-box elements (k₁/k₁', k₂/k₂', s/s').

The left adaptor primer also consists of distinct sequence tracts, designated a_{L} and a_{P}a_{K}, which are used for the second amplification. a_{L} and a_{P} confer functional features for high throughput sequencing e.g. for attachment of the amplificate (amplification product) to a solid surface such as a slide or a bead. The second (adaptor) primer may also comprise template sequences for sequencing primers. a_{K} comprises k₁, which is a sequence element of the K-box.

The reverse or "right" initial primer of both the initial and adaptor primer comprises sequence tracts of similar characteristics, designated kₐ'-k'-box (also designated as k') and p_{C}'. The right adaptor primer consists of the sequence tracts a_{L}' and a_{P}'-a_{K}' (Fig. 1). In general, the "prime" or apostrophe (') indicates that a sequence tract or element has a similar functional characteristic as its non-prime counterpart, but is located on a primer on the other side of the target sequence.

Sequence elements a_{L} and a_{P} are used for sequencing purposes, such as, by way of nonlimiting example, sequencing primer hybridization sites and/or solid support attachment sites. Methods for high-throughput sequencing are well known in the art and include so called "Illumina" bridge PCR-sequencing methods, shown inter alia in US2011045541A1, US2005100900A1, US2002055100A1; pyrosequencing, shown inter alia in US6,274,320, US 7,244,567, US 7,264,929; US 7,323,305 and US 7,575,865; "2 base encoding" technology (US4883750, US5750341) and others. Further relevant methods for high-throughput sequencing and applications are described in the following manuscripts:
- Robustness of Amplicon Deep Sequencing Underlines Its Utility in Clinical Applications. Grossmann V et al. J Mol Diagn. 2013 May 14. doi:pii: S1525-1578(13)00057-3. PMID:23680131.
- Solid-State and Biological Nanopore for Real-Time Sensing of Single Chemical and Sequencing of DNA. Haque F et al. Nano Today. 2013 Feb;8(1):56-74. PMID:23504223.
- Next-generation sequencing - feasibility and practicality in haematology. Kohlmann A et al. Br J Haematol. 2013 Mar;160(6):736-53. doi: 10.1111/bjh.12194. Epub 2013 Jan 7. PMID:23294427.
- Progress in ion torrent semiconductor chip based sequencing. Merriman B et al. Electrophoresis. 2012 Dec;33(23):3397-417. doi: 10.1002/elps.201200424. Erratum in: Electrophoresis. 2013 Feb;34(4):619. PMID:23208921.
- Comparison of next-generation sequencing systems. Liu L et al. J Biomed Biotechnol. 2012;2012:251364. doi: 10.1155/2012/251364. Epub 2012 Jul 5. PMID:22829749.
- Current state-of-art of sequencing technologies for plant genomics research. Thudi M et al. Brief Funct Genomics. 2012 Jan;11(1):3-11. doi: 10.1093/bfgp/elr045. PMID:22345601.
- Integration of next-generation sequencing into clinical practice: are we there yet? Kohlmann A et al. Semin Oncol. 2012 Feb;39(1):26-36. doi: 10.1053/j.seminoncol.2011.11.008. PMID:22289489.

The primers of the invention provide particular sequence elements (K-boxes; designated generally in some embodiments as k and k'), that greatly reduce the likelihood that such contaminations occur and enable the recognition of amplicon contaminations within the sequencing results. The K-box elements are designated k₁, k₁', k₂, k₂', s and s', and must be bioinformatically designed as one single K-box, being selected not to perform mismatches with the 3'ends of the primers employed. However, for clarification of their mode of action the three K-box elements are outlined in the following in detail separately:

### Role of k₁ and k₁' K-box elements and mode of action:

The k₁/k₁' sequences are designed to prevent contamination from previous amplification reactions. As outlined in Fig. 1 the forward primer of the first PCR is composed of (i) a target-specific proportion p_{c}, (ii) and the K-box sequence element k₁ which is specific for each primer set and (iii) a sequence element kₐ. The reverse primer of the first PCR is composed in the same way but in reverse-complement fashion.

A specific k₁ and k₁' element is used for a particular reaction and is varied when the amplification reaction is performed repeatedly. In other words, if a routine diagnostic amplification reaction (e.g. the analysis of T-cell receptor beta rearrangements or the analysis of cancer genes) is performed a plurality of times in the same laboratory, primers using different k_{1/}k₁' elements may be used for each individual experiment until all variations of k₁ and k₁' have been consumed. The 3' end of the second (or adaptor) primer is chosen to hybridize to k₁ or k₁', respectively, along the entire length of k₁ (or k₁'). Thus, pairs of first and second primers are formed, where the "left" adaptor primer hybridizes to sequence tract k₁ which was generated by the "left" initial primer, and the "right" adaptor primer hybridizes to the sequence tract k₁' which was generated by the "right" initial primer. In order to allow full hybridization, the adaptor primer will hybridize not necessarily only to the tract generated by k₁ (or k₁'), but - if k₁ (or k₁') does not provide sufficient length of hybridization tract for the hybridization temperature selected for the reaction - also to a sequence tract adjacent to k₁ (or k₁') on its 5' end, namely kₐ and kₐ' (see Fig. 1).

To give a simple example, if the same amplification reaction is conducted in a laboratory during the working days of a week, five sets of first (initial) and second (adaptor) primers can be employed, with a k₁/k₁' element combination specific for the day, to distinguish the reactions. If Tuesday's reaction were contaminated by a small amount of Monday's first amplification product, the mismatch between the (Monday) k₁ and/or k₁' element of the amplification product (introduced by Monday's first primer set) and the (Tuesday) k₁ and/or k₁' element on the adaptor primer, will inhibit the amplification of the contaminating material. Thus, a matched presence of sequence element k₁ and k₁', respectively, of *both first and second primer* in the same reaction, but mismatched across repeated reactions, inhibits amplification of contaminating sequences.

Both, k₁ and k₁' can be of 1, 2, 3, 4, 5, 6, 7, 8 or more bases in length. As shown in the proof of principle example below (table 9) even a k₁/k₁' sequence of one base reduces contamination. However since the number of permutations is relatively low and the discriminatory power (in the sense of contamination suppression) of a one-base mismatch not as great as that of longer mismatches, k₁ and k₁' elements of greater length, for example 2, 3, 4,5 or 6 have broader utility (Examples are given in tables 19-21).

### Role of k₂ and k₂' K-box elements and mode of action:

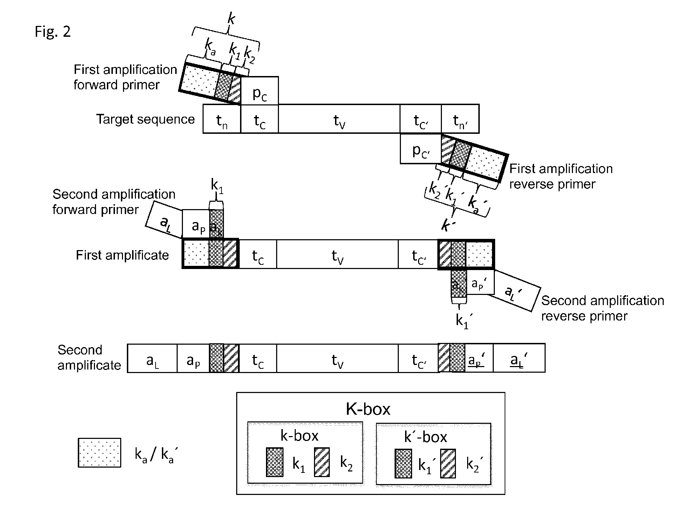

A further K-box element is a sequence element k₂ or k₂', comprised in the sequence tract k or k', respectively, of the initial primer, but not in the corresponding sequence tract in the second amplification (adaptor) primer (Fig. 2). Hence, k₂ and k₂' are characteristic of the initial primer only. In embodiments where k₂ (or k₂') and k₁ (or k₁') sequences are comprised in the initial primer, the k₂ (or k₂') element is downstream (towards the 3' end) from the k₁ (or k₁') sequence element (Fig. 2).

While k₁ and k₁' lead to suppression of contaminations the k₂/k₂' sequences are designed to detect contamination from previous amplification reactions.

Therefore, as in the case of k₁ (or k₁'), the presence of k₂ (or k₂') in specific variation (Examples are provided in Table 19-22) over a plurality of primer sets used at different times or for different samples in the same routine setting helps to detect contaminations and synergistically control the contamination suppression efficacy of k₁ / k₁'.

### Role of s and s' K-box elements and mode of action:

The K-box elements S/S' prevent a possible PCR bias dependent on k₁/k₁' and k₂/k₂' sequences as outlined below.

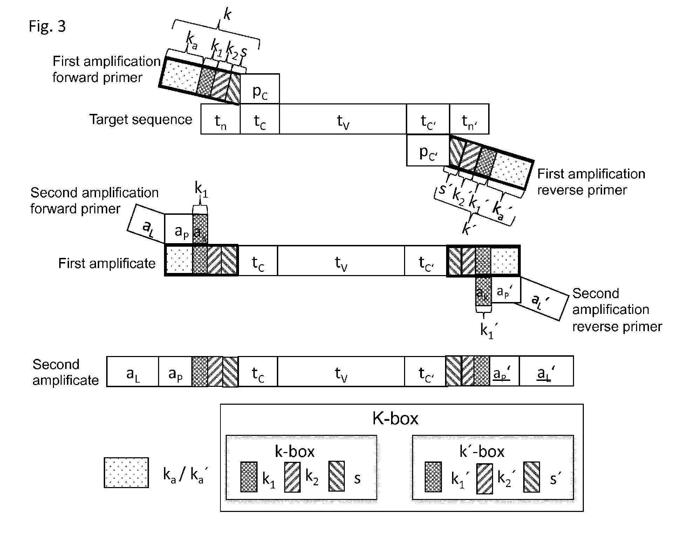

S separates the target-specific left initial primer sequence p_{C} from the sequence tracts k₂ and/or k₁ and S' separates the target-specific right initial primer sequence p_{C}' from k₂' and/or k₁' (see Fig. 3-4). Since k₁/k₁' and k₂/k₂' vary among different primers used in subsequent amplifications, it may well be that some variations of k₂/k₂' and/or k₁/k₁' coincidentally match in their last nucleotides at the 3' end the sequence of the target next to the hybridizing part of the initial primers p_{C} or p_{C}'. Thus, the target sequence-matching tract of the initial primer would be elongated, leading to higher annealing temperatures and thus, possibly, PCR bias.

This problem is even reinforced if - as provided in some embodiments of the present invention - libraries of primer sets are employed, where each library member addresses a different target sequence for use in a multiplex PCR, but each library member carries the same k₁ (and k₁') and k₂ (and k₂') elements. Here, different annealing temperatures might introduce a PCR bias that may significantly skew any quantitative interpretation of the results.

Hence, in some embodiments a short (1, 2, 3 or 4 nucleotides) separator sequence S (S') is introduced into the k/k' sequence tract, immediately upstream of the p_{C}/p_{C'} sequence tract, i.e., at the 3' terminal end of k/k'. s and S' are thus designed to prevent a hybrid formation with the template (target) sequence tₙ/tₙ' which are adjacent to the primer-hybridizing sequence tract t_{C}/t_{C}' as outlined in Fig. 3-4.

### Advantages of using combined k₁, k₂ , s and k'₁, k'_{2,} s' sequence elements:

The three K-box elements work synergistically to achieve the overall goal to prevent PCR-based contamination in applications employing massive parallel sequencing (NGS/HTS) techniques reactions.

Since the k₂/k₂' elements are only present in the first amplification primers the contamination can still be identified in the second amplification product. Thus, k₂/k₂' elements determines and therefore control the contamination suppression efficiency of k₁/k₁'.

Furthermore, S/S' is the K-box family member that solves the problem of a possible PCR bias dependent on k₁/k₁' and k₂/k₂ sequences.

Finally, all three K-box elements together must be bioinformatically designed as one unit and optimized not to form hybrids (e.g. more than 6 bp match within 10 bp) that might lead to mispriming with any primer sequences employed.

### Different aspects of the invention:

According to a first aspect of the invention, a method for amplifying and sequencing a plurality of samples containing target nucleic acid sequences t_{C}-t_{V}-t_{C}' is provided. This method comprises a first amplification step, in which a target nucleic acid sequence is amplified using a first ("initial") primer pair. This initial primer pair is composed of a left (forward) first ("initial") PCR primer having a sequence k-p_{C} composed of two sequence elements k and P_{C} in 5'-3' orientation, and a right (reverse) initial primer having a sequence k'-p_{C}', similarly oriented from 5' to 3'.. The product of the first amplification set is a first amplificate, comprising the target nucleic acid sequence flanked on either side by sequence tracts k and k', respectively (Fig. 1).

The method of the invention further comprises a second amplification step, whereby a first amplificate is re-amplified using a second ("adaptor") primer pair composed of a left second ("adaptor") PCR primer consisting of a sequence 3_{L}-3_{P}-3_{K} composed of the sequence elements a_{L}, a_{P} and a_{K} in 5'-3' orientation and a right second ("adaptor") PCR primer consisting of a sequence 3_{L}'-a_{P}'-3_{K}'. The product of the second amplification set is a second amplificate (Fig. 1).

Within the first primer pair, P_{C} displays the same sequence as the target sequence element t_{C}, P_{C}' is the reverse complimentary sequence to t_{C}'. In other words, P_{C} and P_{C}' are the target-specific primer sequences that hybridize to the target and effect amplification. p_{C} and p_{C'} each independently from one another are 8 to 40 nucleotides in length. In some embodiments, P_{C} and P_{C}' each independently from one another are 8, 10, 12, 14, 16, 18, 20, 21, 22, 23, 24, 25, 26, 27, 28, 29, 30, 32, 34, 36, 38 or 40 nucleotides in length.

Within the first primer pair, k comprises a k-box with the sequence element k₁, and k' comprises a k'-box with a sequence element k₁'. k₁ and k₁' each independently from one another are a sequence 1 to 8 nucleotides in length. A first primer pair and a second primer pair with identical k₁ and k₁' form a set. k₁ and k₁' are the sequence elements that individualize different primer sets from one another. k₁ and k₁' match the first "initial" and the second "adaptor" PCR primer pairs to one another within a set. Thus, k₁ and k₁' in the first initial primer pair match aₖ (k₁) and a_{K}' (k₁') respectively in the second primer pair (Fig. 1).

Sequence tract a_{P}-a_{K} hybridizes to a contiguous sequence on sequence element k, and a_{P}'-a_{K}' hybridizes to a contiguous sequence on sequence element k'. In other words, a_{P}-a_{K} (and its analogue a_{P}'-a_{K}') is the sequence tract on the 3' terminal end of the adaptor primer that recognizes the initial primer.

k and k' can be of any length that fits their general purpose, but will generally be within the usual length of a primer target sequence, i.e. k and k' will be generally each independently from one another a sequence 10 to 40 nucleotides in length, in many embodiments from about 15 to 30 nucleotides in length.

a_{L} and a_{L}' and also a_{P} and a_{P'} independently from one another can be any sequence, that fits the general purpose of providing a sequence useful for sequencing the second amplificate, for example by providing a sequencing primer annealing target and/or a sequence for attaching the second amplificate to a chip or bead or any other surface-bound structure as may be useful in high-throughput ("next generation") sequencing.

In some embodiments, a_{L} and 3_{L}' are 5, 10, 15, 20, 25, 30, 35, 40, 45, 50, 55, 60, 65 or 70 nucleotides in length.

t_{V} is a variable region within said target nucleic acid sequence.

In some embodiments, the left initial primer, the right initial primer, the left adaptor primer and/or the right adaptor primer are characterized by a nuclease resistant nucleotide or a nuclease resistant internucleosidic bond on the 3' terminus of the primers. In other words, the 3' end of the primer is protected against 3' exonuclease digestion by providing bonds that inhibit or resist the exonuclease activity.

In some embodiments, the nuclease resistant internucleosidic bond is a phosphorothioate bond. In some embodiments, the nuclease resistant nucleotide is a 2-O-methylated ribonucleotide. In some embodiments, the nuclease resistant nucleotide is an LNA building block (a 2'O, 4'C-methylene bridged RNA building block). In some embodiments, the nuclease resistant nucleotide is a 2-F-deoxyribonucleotide. In some embodiments, the nuclease resistant nucleotide is a 2-propyne-deoxyribonucleotide.

In some embodiments, the nuclease resistant nucleotide or nuclease resistant internucleosidic bond is the last internucleosidic bond counting from the 3' terminus of said primer(s). In some embodiments, the nuclease resistant nucleotide or nuclease resistant internucleosidic bond is located on position -1, -2, -3, and/or-4 counting from the 3' terminus of said primer(s). In some embodiments, the nuclease resistant nucleotide or nuclease resistant internucleosidic bond are located at position - 1 and - 2, in some embodiments at position -1 and -2 and - 3, or in some embodiments at position -1 and -2 and - 3 and -4.

Adjacent to the target nucleic acid sequence t_{C}-t_{V}-t_{C}', sequence elements tₙ and tₙ' are located in 5' and 3' position, respectively. In other words, the target structure can be described as tₙ-t_{C}-t_{V}-t_{C}'-tₙ' (Fig. 3-4). k comprises a sequence element S on its 3' terminus and k' comprises a sequence element s' on its 3' terminus, wherein s and S' are mismatch sequences selected not to form a continuous hybrid sequence with sequence element tₙ and tₙ' and s and S' are independently 1, 2, 3 or 4 nucleotides in length. As described in detail above the effect of this feature is to avoid an inadvertent rise of the annealing temperature of p_{C} and p_{C}' on the target in some primers dependent on k and/or k' sequences. This feature helps to avoid PCR bias (Fig. 3-4).

k comprises a sequence element k₂ 3'-terminal to sequence element k₁, and k' comprises a sequence element k₂' 3'-terminal to sequence element k₁' (Fig. 2). k₂ and k₂' each independently from one another are 1, 2, 3, 4 or 5 nucleotides in length. k₂ and k₂' serve to individualize the first primer pair of the set from other first (initial) primers. k₂ and k₂' have no complementary sequence elements on the second ("adaptor") primers. The second primers k₁ and k₁' have complementary sequences to the first primers within one primer set.

The primer set uses all three elements k₁/k₁'_{,} k₂/k₂' and s/s' (Fig. 3). Thus k comprises a 3'-terminal sequence k₁-k₂-s, and k' comprises an 3'-terminal sequence k₁'-k₂'-s'. k comprises a 3'-terminal sequence k₁-k₂-s, and k' comprises a 3'-terminal sequence k₁'-k₂'-s' and the first and/or second primer pairs have phosphorothiolated moieties on the last 1, 2, 3 or 4 internucleotide linkages at their 3'terminal end.

A method for sequencing a target sequence t_{C}-t_{V}-t_{C}' comprises the steps of
a. amplifying the target sequence by a method as outlined above, and
b. sequencing the second amplificate with a sequence stretch including sequence elements k and/or k', yielding a set of readout sequences.

The method for sequencing a target sequence further comprises the steps of
c. aligning each member of said set of readout sequences to sequence element k and/or k' comprised in the initial primer sequence, respectively, and
d. assigning a measure 0 or 1 of contamination to said set of readout sequences, wherein complete alignment of each member of said set of readout sequences to said sequence element k and/or k' is assigned a 0 measure of contamination (= no contamination), and incomplete alignment of said set of readout sequences to said sequence element k and/or k' is assigned a measure 1 of contamination (= is contaminated).

In other words, the method for sequencing a target sequence includes a step of validation or quality control, wherein all sequences obtained are checked for the presence of identifier sequences k₁, k₁', and/or k₂, k₂' (k₁, k₁' can be relevant as identifier, since k₁, k₁' can be partially degraded in the second PCR by proof reading polymerases as outlined in detail in the proof of principle examples). Unexpected identifier sequences, or unexpected combinations thereof, are regarded as contamination.

According to the invention, a method for sequencing a plurality of samples containing target nucleic acid sequences t_{C}-t_{V}-t_{C}' is provided, comprising the steps amplification and sequencing laid out above according to any of the proposed alternative aspects or embodiments of the invention, wherein for each sample, a different set of primers is used, the difference being in different sequence elements k₁, k₁', k₂ and k₂', or combinations thereof. In other words, the method comprises the steps of providing a set of primers for each sample of said plurality of samples, each set of primers comprising a pair of initial PCR primers comprising a left initial PCR primer having a sequence k-p_{C} and a right initial primer having a sequence and k'-p_{C}', and a pair of adaptor PCR primers comprising a left adaptor PCR primer consisting of a sequence a_{L}-a_{P}-a_{K} and a right adaptor PCR primer consisting of a sequence a_{L}'-a_{P}'-a_{K}'.

Within the pair of initial PCR primers, k comprises a sequence element k₁, and k' comprises a sequence element k₁'. k₁ and k₁' each independently from one another are a sequence of 1 to 8 nucleotides in length. k₁ and k₁' are the sequence elements that individualize different primer sets from one another. k₁ and k₁' match the first "initial" and the second "adaptor" PCR primer pairs to one another within a set. Thus, as a matching sequence to k₁ and k₁' in the first initial primer pair, aₖ is the same sequence as sequence element k, and a_{K}' is the same sequence as sequence element k₁' (Fig. 1-4).

All sequence element designators have the definition as laid out above.

According to the method of the invention, for all sets of primers, all sequence elements p_{C} are the same; all sequence elements p_{C}' are the same; all sequence elements 3_{L}-a_{P} are the same; all sequence elements a_{L}'-a_{P}' are the same; however each set of primers is characterized by a different combination of k₁ and k₁' from any other set of primers. The method further comprises the step of obtaining a set of readout sequences from each of the samples by the steps of
i. effecting a first amplification step, whereby the target nucleic acid sequence is amplified using said set of initial PCR primers, yielding a first amplificate;
ii. effecting a second amplification step, whereby the first amplificate is amplified using said set of adaptor PCR primers, yielding a second amplificate;
iii. sequencing the second amplificate, yielding a set of readout sequences;
iv. aligning said set of readout sequences to sequence elements k and k' comprised in said set of initial primers, and
v. assigning a measure 0 or 1 of contamination to said set of readout sequences, wherein complete alignment of each member of said set of readout sequences to said sequence element k or k' is assigned a measure 0 (= no contamination), and incomplete alignment of said set of readout sequences to said sequence element k or k' is assigned a measure 1 of contamination (= is contaminated).

The target nucleic acid can be described as such that adjacent to the target nucleic acid sequence t_{C}-t_{V}-t_{C}', sequence elements tₙ and tₙ' are located in 5' and 3' position, respectively, and for each set of primers, k comprises a sequence element S on its 3' terminus and k' comprises a sequence element s' on its 3' terminus, wherein s and S' are mismatch sequences selected not to form a continuous hybrid sequence with sequence element tₙ and tₙ' , and s and s' are independently 1, 2, 3 or 4 nucleotides in length (Fig. 3-4).

k comprises a sequence element k₂ 3'-terminal to sequence element k₁, and k' comprises a sequence element k₂' 3'-terminal to sequence element k₁', k₂ and k₂' each independently from one another are 1, 2, 3, 4 or 5 nucleotides in length, and each set of primers is characterized by a different combination of k₂ and k₂' from any other set of primers (Fig. 3).

In some embodiments, each k and k' in each set of primers are selected to have a hybridizing temperature differing less than 6°C from the hybridizing temperature of any other k and k' comprised in said set of sequences. Selection of inter-primer hybridizing tracts of similar annealing temperature reduces inter-reaction PCR bias.

According to another aspect of the invention, a collection of sets of primers for use in a method for sequencing samples containing target nucleic sequences t_{C}-t_{V}-t_{C}' is provided. Each set of primers of said collection comprises a set of initial PCR primers comprising a left initial PCR primer having a sequence k-p_{C} and a right initial primer having a sequence and k'-p_{C}', and a set of adaptor PCR primers comprising a left adaptor PCR primer consisting of a sequence a_{L}-a_{P}-a_{K} and a right adaptor PCR primer consisting of a sequence a_{L}'-a_{P}'-a_{K}'. All sequence elements have the definition as laid out above.

For all sets of primers comprised within said collection, all sequence elements P_{C} are the same; all sequence elements p_{C}' are the same; all sequence elements a_{P} are the same; all sequence elements a_{P'} are the same; and each set of primers is characterized by a different combination of k₁ and k₁' from any other set of primers.

In the collection of primers, adjacent to said target nucleic acid sequence t_{C}-t_{V}-t_{C}', sequence elements tₙ and tₙ' are located in 5' and 3' position, respectively, and for each set of primers, k comprises a sequence element S on its 3' terminus and k' comprises a sequence element s' on its 3' terminus, wherein s and S' are mismatch sequences selected not to form a continuous hybrid sequence with sequence element tₙ and tₙ', and s and S' are independently 1, 2, 3 or 4 nucleotides in length.

In the collection of primers, k comprises a sequence element k₂ 3'-terminal to sequence element k₁, and k' comprises a sequence element k₂' 3'-terminal to sequence element k₁', k₂ and k₂' each independently from one another are 1, 2, 3, 4 or 5 nucleotides in length, and each set of primers is characterized by a different combination of k₂ and k₂' from any other set of primers.

According to yet another aspect of the invention, a primer library comprising a plurality of collections of sets of primers according to the invention is provided, wherein each collection is characterized by a different combination of P_{C} and p_{C}' from any other set of primers; in other words: each collection is characterized by a specific pc and/or p_{c}' from any other collection of primers.

If a collection of primers has been defined herein as a plurality of sets having the same target nucleic acid sequence, but different discriminating or identifying sequence elements k₁, k₁', k₂ and k₂', then a library member may be defined as a multiplex-collection, whereby all primers of a library have the same identifying sequence elements k₁, k₁', and/ or k₂, k₂', but different targets. In other words, a library member may be defined as a plurality of sets each having the same identifying sequence elements k₁, k₁', and/ or k₂, k₂', but different targets. The library members can thus be used together, within the same multiplex PCR, and different library members (discriminated by different K-boxes) will be used in repeated routine PCR/sequencing rounds.

According to yet another aspect of the invention, a method for sequencing a plurality of samples containing a plurality of target nucleic acid sequences t_{C1}-t_{V1}-t_{C1}', t_{C2}-t_{V2}-t_{C2}', t_{C3}-t_{V3}-t_{C3}', ... t_{Ci}-t_{Vi}-t_{Ci}' is provided, wherein a plurality of sets of primers is employed, each set of primers comprising a pair of initial PCR primers comprising a left initial PCR primer having a sequence k-p_{C} and a right initial primer having a sequence and k'-p_{C}', and a pair of adaptor PCR primers comprising a left adaptor PCR primer consisting of a sequence a_{L}-a_{P}-a_{K} and a right adaptor PCR primer consisting of a sequence a_{L}'-a_{P}'-a_{K}'- Within said pair of initial PCR primers, k comprises a sequence element k₁, and k' comprises a sequence element k₁'. k₁ and k₁' each independently from one another are a sequence 1 to 8 nucleotides in length. k₁ and k₁' are the sequence elements that individualize different primer sets from one another. k₁ and k₁' match the first "initial" and the second "adaptor" PCR primer pairs to one another within a set. Thus, as a matching sequence to k₁ and k₁' in the first initial primer pair, aₖ is the same sequence as sequence element k₁, and a_{K}' is the same sequence as sequence element k₁'.

All sequence elements have the definition as laid out above.

According to this alternative aspect of the method of the invention, within the plurality of sets of primers, all sequence elements k₁ and k₁' are the same; all sequence elements a_{L}-a_{P} are the same; all sequence elements a_{L}'- a_{P}' are the same; however each set of primers is characterized by a different combination of P_{C} and p_{C'} from any other set of primers.

In one alternative, a plurality of sets of primers is provided having left first "initial" primers having sequences k-p_{C1}, k-p_{C2}, k-P_{C3}, k-p_{Cj}, and right first "initial" primers having sequences k-p_{C}'₁, k-P_{C}'₂, k-P_{C}'₃, k-p_{C}'ⱼ, wherein all sequence elements k₁ and k₁' are the same; all sequence elements a_{L}-a_{P} are the same; all sequence elements a_{L}'- a_{P}' are the same. The k and k' tracts comprise sequence elements k₁ (k₁') and/or k₂ (k₂') and/or s (s') (Fig. 1-4).

To demonstrate the validity and power of the present invention, a PCR-based analysis of T-cell receptor (TCR) gene rearrangements was performed.

In general, the use of a nested PCR approach for TCR analyses has the advantage that the initial PCR with gene-specific TCR primers requires only a few PCR cycles minimizing PCR-generated bias, and thereafter the first amplificate is amplified evenly with the adaptor specific primers by a further PCR step. Furthermore, different adaptors suitable for different NGS platforms can be added by the second PCR.

In one embodiment, sequences for the target-binding tract of left (P_{C}) initial primer are those given in table 1, and right (P_{C}') initial primer sequences are those given in table 2. The primers in table 1 and 2 were optimized in two aspects: 1) to have a similar annealing temperature and 2) to minimize self priming.

**Table 1: Sequences for amplification of TCR variable regions for TCR-beta repertoire characterisation (left initial primer, target-specific region P_{C} and an example for S)**

| **Segment** | **s** | **Seq Id** | **P_{c}** |
|---|---|---|---|
| V2 | GG | 1 | AATTTCACTCTGAAGATCCGGTCCACAA |
| V3-1 | GG | 2 | CACTTAAATCTTCACATCAATTCCCTGG |
| V4-1 | GG | 3 | TTAAACCTTCACCTACACGCCCTGC |
| V4-2/3 | GG | 4 | TTATTCCTTCACCTACACACCCTGC |
| V5-1 | GG | 5 | AACTCTGAGATGAATGTGAGCACCTTG |
| V5-3 | GG | 6 | TGCTCTGAGATGAATGTGAGTGCCTTG |
| V5-4 / 5 / 6 / 7 / 8 | GG | 7 | AGCTCTGAGCTGAATGTGAACGCCTTG |
| V6-1 | GG | 8 | TCGCTCAGGCTGGAGTCGGCTG |
| V6-2/3 | GG | 9 | ATTTTCCTGGGGTTGGAGTCGGCTG |
| V6-4 | GG | 10 | TTCCCCCTCACGTTGGCGTCTGCTG |
| V6-5 | GG | 11 | CCGCTCAGGCTGCTGTCGGCTG |
| V6-6 | GG | 12 | TTCCCGCTCAGGCTGGAGTTGGCTG |
| V6-7 | GG | 13 | TTCCCCCTCAAGCTGGAGTCAGCTG |
| V6-8 | GG | 14 | TTCCCACTCAGGCTGGTGTCGGCTG |
| V6-9 | GG | 15 | TTCCCGCTCAGGCTGGAGTCAGCTG |
| V7-1 | GG | 16 | TCCACTCTGAAGTTCCAGCGCACAC |
| V7-2 | GG | 17 | TCCACTCTGACGATCCAGCGCACAC |
| V7-3 | GG | 18 | TCTACTCTGAAGATCCAGCGCACAG |
| V7-4 | GG | 19 | TCCACTCTGAAGATCCAGCGCACAG |
| V7-6 | GG | 20 | TCCACTCTGACGATCCAGCGCACAG |
| V7-7 | GG | 21 | TCCACTCTGACGATTCAGCGCACAG |
| V7-8 | GG | 22 | TCCACTCTGAAGATCCAGCGCACAC |
| V7-9 | GG | 23 | TCCACCTTGGAGATCCAGCGCACAG |
| V9 | GG | 24 | CACTCTGAACTAAACCTGAGCTCTCTG |
| V10-1 | GG | 25 | CTCCCCCTCACTCTGGAGTCTGCTG |
| V10-2 | GG | 26 | TTCCCCCTCACTCTGGAGTCAGCTA |
| V10-3 | GG | 27 | TTCCTCCTCACTCTGGAGTCCGCTA |
| V11-1/3 | GG | 28 | TCCACTCTCAAGATCCAGCCTGCAG |
| V11-2 | GG | 29 | TCCACTCTCAAGATCCAGCCTGCAA |
| V12-3 / 4 / 5 | GG | 30 | TCCACTCTGAAGATCCAGCCCTCAG |
| V13 | GG | 31 | CATTCTGAACTGAACATGAGCTCCTTGG |
| V14 | GG | 32 | TCTACTCTGAAGGTGCAGCCTGCAG |
| V15 | GG | 33 | AACTTCCAATCCAGGAGGCCGAACA |
| V16 | GG | 34 | TGTAGCCTTGAGATCCAGGCTACGA |
| V17 | GG | 35 | TCTTCCACGCTGAAGATCCATCCCG |
| V18 | GG | 36 | AGCATCCTGAGGATCCAGCAGGTAG |
| V19 | GG | 37 | TTTCCTCTCACTGTGACATCGGCCC |
| V20-1 | GG | 38 | TTGTCCACTCTGACAGTGACCAGTG |
| V23-1 | GG | 39 | TGCAGCCTGGCAATCCTGTCCTCAG |
| V24-1 | GG | 40 | TTCTCCCTGTCCCTAGAGTCTGCCAT |
| V25-1 | GG | 41 | TTTCCCCTGACCCTGGAGTCTGCCA |
| V27 | GG | 42 | TTCCCCCTGATCCTGGAGTCGCCCA |
| V28 | GG | 43 | TTCTCCCTGATTCTGGAGTCCGCCA |
| V29-1 | GG | 44 | ACATTCTCAACTCTGACTGTGAGCAACA |
| V30 | GG | 45 | CGGCAGTTCATCCTGAGTTCTAAGAAGC |

**Table 2: Sequences for amplification of TCR joining regions in TCR-beta repertoire characterisation (right initial primer, target-specific region p_{C} 'and an example for s')**

| **Segment** | **s**' | **Seq Id** | **p_{C}'** |
|---|---|---|---|
| **J1-1** | **TA** | 46 | TTACCTACAACTGTGAGTCTGGTGCC |
| **J1-2** | **TA** | 47 | CCTACAACGGTTAACCTGGTCCCCG |
| **J1-3** | **TA** | 48 | CCTACAACAGTGAGCCAACTTCCCT |
| **J1-4** | **TA** | 49 | CCCAAGACAGAGAGCTGGGTTCCACT |
| **J1-5** | **TA** | 50 | TTACCTAGGATGGAGAGTCGAGTCC |
| **J1-6** | **TA** | 51 | CCTGTCACAGTGAGCCTGGTCCCGT |
| **J2-1** | **TA** | 52 | CCTAGCACGGTGAGCCGTGTCCC |
| **J2-2** | **TA** | 53 | TTACCCAGTACGGTCAGCCTAGAGC |
| **J2-3** | **TA** | 54 | AGCACTGTCAGCCGGGTGCCTGG |
| **J2-4** | **TA** | 55 | AGCACTCAGAGCCGGGTCCCGGC |
| **J2-5** | **TA** | 56 | CCGAGCACCAGGAGCCGCGTGC |
| **J2-6** | **TA** | 57 | AGCACGGTCAGCCTGCTGCCGGC |
| **J2-7** | **TA** | 58 | GTGACCGTGAGCCTGGTGCCCGG |

In one embodiment the sequence element kₐ is one of those given in table 3.

**Table 3: Examples of sequence element kₐ in the left (forward) first amplification primer.**

| **SEQ ID** | **Sequence** |
|---|---|
| 59 | ATCTACACTCTTTCCCTACACGACGCTCTTCCGATCT |
| 60 | TCTACACTCTTTCCCTACACGACGCTCTTCCGATCT |
| 61 | CTACACTCTTTCCCTACACGACGCTCTTCCGATCT |
| 62 | TACACTCTTTCCCTACACGACGCTCTTCCGATCT |
| 63 | ACACTCTTTCCCTACACGACGCTCTTCCGATCT |
| 64 | CACTCTTTCCCTACACGACGCTCTTCCGATCT |
| 65 | ACTCTTTCCCTACACGACGCTCTTCCGATCT |
| 66 | CTCTTTCCCTACACGACGCTCTTCCGATCT |
| 67 | TCTTTCCCTACACGACGCTCTTCCGATCT |
| 68 | CTTTCCCTACACGACGCTCTTCCGATCT |
| 69 | TTTCCCTACACGACGCTCTTCCGATCT |
| 70 | TTCCCTACACGACGCTCTTCCGATCT |
| 71 | TCCCTACACGACGCTCTTCCGATCT |
| 72 | CCCTACACGACGCTCTTCCGATCT |
| 73 | CCTACACGACGCTCTTCCGATCT |
| 74 | CTACACGACGCTCTTCCGATCT |
| 75 | TACACGACGCTCTTCCGATCT |
| 76 | ACACGACGCTCTTCCGATCT |
| 77 | CACGACGCTCTTCCGATCT |
| 78 | ACGACGCTCTTCCGATCT |
| 79 | CGACGCTCTTCCGATCT |
| 80 | GACGCTCTTCCGATCT |
| 81 | ACGCTCTTCCGATCT |
| 82 | CGCTCTTCCGATCT |
| 83 | GCTCTTCCGATCT |
| 84 | CTCTTCCGATCT |
| 85 | TCTTCCGATCT |

In one embodiment the sequence element kₐ' is one of those given in table 4.

**Table 4: Examples of sequence element kₐ' in the right (reverse) first amplification primer.**

| **SEQ ID** | **Sequence** |
|---|---|
| 86 | TACGAGATGTGACTGGAGTTCAGACGTGTGCTCTTCCGATCT |
| 87 | ACGAGATGTGACTGGAGTTCAGACGTGTGCTCTTCCGATCT |
| 88 | CGAGATGTGACTGGAGTTCAGACGTGTGCTCTTCCGATCT |
| 89 | GAGATGTGACTGGAGTTCAGACGTGTGCTCTTCCGATCT |
| 90 | AGATGTGACTGGAGTTCAGACGTGTGCTCTTCCGATCT |
| 91 | GATGTGACTGGAGTTCAGACGTGTGCTCTTCCGATCT |
| 92 | ATGTGACTGGAGTTCAGACGTGTGCTCTTCCGATCT |
| 93 | TGTGACTGGAGTTCAGACGTGTGCTCTTCCGATCT |
| 94 | GTGACTGGAGTTCAGACGTGTGCTCTTCCGATCT |
| 95 | TGACTGGAGTTCAGACGTGTGCTCTTCCGATCT |
| 96 | GACTGGAGTTCAGACGTGTGCTCTTCCGATCT |
| 97 | ACTGGAGTTCAGACGTGTGCTCTTCCGATCT |
| 98 | CTGGAGTTCAGACGTGTGCTCTTCCGATCT |
| 99 | TGGAGTTCAGACGTGTGCTCTTCCGATCT |
| 100 | GGAGTTCAGACGTGTGCTCTTCCGATCT |
| 101 | GAGTTCAGACGTGTGCTCTTCCGATCT |
| 102 | AGTTCAGACGTGTGCTCTTCCGATCT |
| 103 | GTTCAGACGTGTGCTCTTCCGATCT |
| 104 | TTCAGACGTGTGCTCTTCCGATCT |
| 105 | TCAGACGTGTGCTCTTCCGATCT |
| 106 | CAGACGTGTGCTCTTCCGATCT |
| 107 | AGACGTGTGCTCTTCCGATCT |
| 108 | GACGTGTGCTCTTCCGATCT |
| 109 | ACGTGTGCTCTTCCGATCT |
| 110 | CGTGTGCTCTTCCGATCT |
| 111 | GTGTGCTCTTCCGATCT |
| 112 | TGTGCTCTTCCGATCT |
| 113 | GTGCTCTTCCGATCT |
| 114 | TGCTCTTCCGATCT |
| 115 | GCTCTTCCGATCT |
| 116 | CTCTTCCGATCT |
| 117 | TCTTCCGATCT |

In one embodiment sequences of the adaptor primes are those given in table 5.

**Table 5: Examples for sequences a_{L}-a_{P} of the left adaptor primer**

| **SEQ ID** | **Sequence** |
|---|---|
| 118 | AATGATACGGCGACCACCGAGATCTACACTCTTTCCCTACACGACGCTCTTCCGATCT |
| 119 | AATGATACGGCGACCACCGAGATCTACACTCTTTCCCTACACGACGCTCTTCCGATC |
| 120 | AATGATACGGCGACCACCGAGATCTACACTCTTTCCCTACACGACGCTCTTCCGAT |
| 121 | AATGATACGGCGACCACCGAGATCTACACTCTTTCCCTACACGACGCTCTTCCGA |
| 122 | AATGATACGGCGACCACCGAGATCTACACTCTTTCCCTACACGACGCTCTTCCG |
| 123 | AATGATACGGCGACCACCGAGATCTACACTCTTTCCCTACACGACGCTCTTCC |
| 124 | AATGATACGGCGACCACCGAGATCTACACTCTTTCCCTACACGACGCTCTTC |
| 125 | AATGATACGGCGACCACCGAGATCTACACTCTTTCCCTACACGACGCTCTT |
| 126 | AATGATACGGCGACCACCGAGATCTACACTCTTTCCCTACACGACGCTCT |
| 127 | AATGATACGGCGACCACCGAGATCTACACTCTTTCCCTACACGACGCTC |
| 128 | AATGATACGGCGACCACCGAGATCTACACTCTTTCCCTACACGACGCT |
| 129 | AATGATACGGCGACCACCGAGATCTACACTCTTTCCCTACACGACGC |
| 130 | AATGATACGGCGACCACCGAGATCTACACTCTTTCCCTACACGACG |
| 131 | AATGATACGGCGACCACCGAGATCTACACTCTTTCCCTACACGAC |
| 132 | AATGATACGGCGACCACCGAGATCTACACTCTTTCCCTACACGA |
| 133 | AATGATACGGCGACCACCGAGATCTACACTCTTTCCCTACACG |
| 134 | AATGATACGGCGACCACCGAGATCTACACTCTTTCCCTACAC |
| 135 | AATGATACGGCGACCACCGAGATCTACACTCTTTCCCTACA |
| 136 | AATGATACGGCGACCACCGAGATCTACACTCTTTCCCTAC |
| 137 | AATGATACGGCGACCACCGAGATCTACACTCTTTCCCTA |
| 138 | AATGATACGGCGACCACCGAGATCTACACTCTTTCCCT |
| 139 | AATGATACGGCGACCACCGAGATCTACACTCTTTCCC |
| 140 | AATGATACGGCGACCACCGAGATCTACACTCTTTCC |
| 141 | AATGATACGGCGACCACCGAGATCTACACTCTTTC |
| 142 | AATGATACGGCGACCACCGAGATCTACACTCTTT |
| 143 | AATGATACGGCGACCACCGAGATCTACACTCTT |
| 144 | AATGATACGGCGACCACCGAGATCTACACTCT |
| 145 | AATGATACGGCGACCACCGAGATCTACACTC |
| 146 | AATGATACGGCGACCACCGAGATCTACACT |
| 147 | AATGATACGGCGACCACCGAGATCTACA |
| 148 | AATGATACGGCGACCACCGAGATCTAC |
| 149 | AATGATACGGCGACCACCGAGATCTA |

In one embodiment sequences of the adaptor primer are those given in table 6.

**Table 6: Examples for sequences a_{L}'-a_{P}' of the right adaptor primer.**

| **SEQ ID** | **Sequence** |
|---|---|
| 150 | CAAGCAGAAGACGGCATACGAGATGTGACTGGAGTTCAGACGTGTGCTCTTCCGATCT |
| 151 | CAAGCAGAAGACGGCATACGAGATGTGACTGGAGTTCAGACGTGTGCTCTTCCGATC |
| 152 | CAAGCAGAAGACGGCATACGAGATGTGACTGGAGTTCAGACGTGTGCTCTTCCGAT |
| 153 | CAAGCAGAAGACGGCATACGAGATGTGACTGGAGTTCAGACGTGTGCTCTTCCGA |
| 154 | CAAGCAGAAGACGGCATACGAGATGTGACTGGAGTTCAGACGTGTGCTCTTCCG |
| 155 | CAAGCAGAAGACGGCATACGAGATGTGACTGGAGTTCAGACGTGTGCTCTTCC |
| 156 | CAAGCAGAAGACGGCATACGAGATGTGACTGGAGTTCAGACGTGTGCTCTTC |
| 157 | CAAGCAGAAGACGGCATACGAGATGTGACTGGAGTTCAGACGTGTGCTCTT |
| 158 | CAAGCAGAAGACGGCATACGAGATGTGACTGGAGTTCAGACGTGTGCTCT |
| 159 | CAAGCAGAAGACGGCATACGAGATGTGACTGGAGTTCAGACGTGTGCTC |
| 160 | CAAGCAGAAGACGGCATACGAGATGTGACTGGAGTTCAGACGTGTGCT |
| 161 | CAAGCAGAAGACGGCATACGAGATGTGACTGGAGTTCAGACGTGTGC |
| 162 | CAAGCAGAAGACGGCATACGAGATGTGACTGGAGTTCAGACGTGTG |
| 163 | CAAGCAGAAGACGGCATACGAGATGTGACTGGAGTTCAGACGTGT |
| 164 | CAAGCAGAAGACGGCATACGAGATGTGACTGGAGTTCAGACGTG |
| 165 | CAAGCAGAAGACGGCATACGAGATGTGACTGGAGTTCAGACGT |
| 166 | CAAGCAGAAGACGGCATACGAGATGTGACTGGAGTTCAGACG |
| 167 | CAAGCAGAAGACGGCATACGAGATGTGACTGGAGTTCAGAC |
| 168 | CAAGCAGAAGACGGCATACGAGATGTGACTGGAGTTCAGA |
| 169 | CAAGCAGAAGACGGCATACGAGATGTGACTGGAGTTCAG |
| 170 | CAAGCAGAAGACGGCATACGAGATGTGACTGGAGTTCA |
| 171 | CAAGCAGAAGACGGCATACGAGATGTGACTGGAGTTC |
| 172 | CAAGCAGAAGACGGCATACGAGATGTGACTGGAGTT |
| 173 | CAAGCAGAAGACGGCATACGAGATGTGACTGGAGT |
| 174 | CAAGCAGAAGACGGCATACGAGATGTGACTGGAG |
| 175 | CAAGCAGAAGACGGCATACGAGATGTGACTGGA |
| 176 | CAAGCAGAAGACGGCATACGAGATGTGACTGG |
| 177 | CAAGCAGAAGACGGCATACGAGATGTGACTG |
| 178 | CAAGCAGAAGACGGCATACGAGATGTGACT |
| 179 | CAAGCAGAAGACGGCATACGAGATGTGAC |
| 180 | CAAGCAGAAGACGGCATACGAGATGTGA |
| 181 | CAAGCAGAAGACGGCATACGAGATGTG |
| 182 | CAAGCAGAAGACGGCATACGAGATGT |

Wherever alternatives for single separable features are laid out herein as "embodiments", it is to be understood that such alternatives may be combined freely to form discrete embodiments of the invention disclosed herein.

The invention is further illustrated by the following examples and figures, from which further embodiments and advantages can be drawn. These examples are meant to illustrate the invention but not to limit its scope.

### Short description of the figures

- Fig. 1: shows the primers, target and first and second amplificate of the method of the invention, wherein the sequence tract k comprises a sequence element k₁ and the sequence tract k' comprises a sequence element k₁'. Reverse complementary sequence tracts are underlined.
- Fig. 2: shows the primers, target and first and second amplificate of the method of the invention, wherein the sequence tract k, in addition to k₁, comprises a sequence element k₂. and the sequence tract k', in addition to k₁', comprises a sequence element k₂.'. Reverse complementary sequence tracts are underlined.
- Fig. 3: shows the primers, target and first and second amplificate of the method of the invention, wherein the sequence tract k, in addition to k₁ and k₂ comprises a sequence element s and the sequence tract k', in addition to k₁' and k₂.', comprises a sequence element s'. Reverse complementary sequence tracts are underlined.

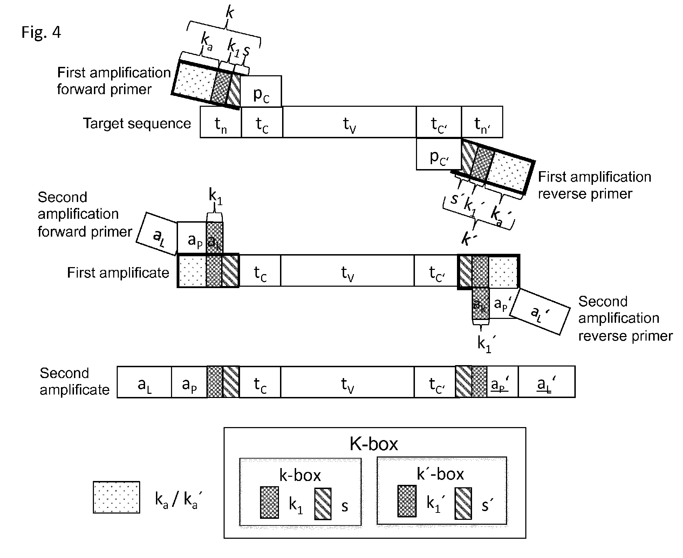

- Fig. 4: shows the primers, target and first and second amplificate of the method of the invention, wherein the sequence tract k, in addition to k₁ comprises a sequence element s and the sequence tract k', in addition to k₁' comprises a sequence element s'. Reverse complementary sequence tracts are underlined.

### Examples:

Proof of principle experiments for the K-box elements (k₁/k₁', k₂/k_{2'} and s/s').

In the proof of principle experiments k₁/k₁' with a lower case k are together also termed K₁ with a capital letter and k₂/k₂' sequence tracts are thereafter also termed K₂. Furthermore, s₁/s₁' sequence tracts are thereafter also termed S. In general, the "prime" or apostrophe (') indicates that a sequence tract or element has a similar functional characteristic as its non-prime counterpart, but is located on a primer on the other side of the target sequence.

### Example 1: Proof of principle for K₁ function.

The basic experimental layout to demonstrate contamination suppression is outlined below:
1) PCR products of the first amplification were defined as a 100% contamination, and were used as template for the second amplification. In order to demonstrate the function and effectiveness of K₁ sequence tracts to suppress this contamination, K₁ mismatches of different length (N = 1, 2, 3, 4, 6 bp) between primers of the first and second (nested) PCR amplification were investigated. Furthermore, effects on contamination suppression, (i) employing polymerases with and without proofreading activity and (ii) primers with and without phosphorothioate bonds were analysed.
2) For comparison - to simulate the situation without contamination suppression - PCRs employing primers with completely matching K₁ sequences were performed.
3) From the PCRs of (1) and (2) the quantities of the PCR products were determined and normalized as described more detailed below. Replicates were performed for all experiments and the mean and standard deviation of PCR product quantity was calculated to obtain statistical reliable results.

If a reamplification with K₁ sequence tracts that mismatch between the first and the second amplification primers showed no PCR product after the second amplification, this was regarded as a complete suppression of the contamination from the primary amplification.

If a reamplification with K₁ sequence tracts that mismatch between the first and the second amplification primers showed PCR products after the second amplification, this was regarded as an incomplete suppression of the contamination from the primary amplification.

### Detailed description of the methods:

PCR was performed using a DNA thermal cycler (PE 9700, Perkin Elmer, Rodgau, Germany). As template for first amplification reactions 100 ng DNA from the T-cell lymphoma cell line Peer was applied, which carries a known TCR-beta gene rearrangement employing the V-4 and J-2-.1 segments.

The initial primers used for the first round of amplification comprised in order from 5' to 3' end a sequence tract k and a sequence tract P_{C} (Fig. 1). Sequence tract P_{C} provided target specificity, while k provided a non-target-specific sequence tract, parts or all of which can be used for hybridization of a second "adaptor" primer. The left initial PCR primers had a sequence k-p_{C} with the matching sequence p_{C} to the V-4 segment (Seq Id 183; TTATTCCTTCACCTACACACCCTGC), whereas the right initial primers which had the sequence and k'-p_{C}' with the matching sequence P_{C}' (Seq ID 184; AGCACTGTCAGCCGGGTGCCTGG) to the J-2.1 segment.

The 3' end of the k-box of forward initial primers had the sequence element S with the two nucleotides "GG", whereas the 3' end of the k'-box of the initial reverse primer had a sequence element s' with two nucleotides "TA".

Furthermore, the k-box of the forward initial primers had a sequence element kₐ (Seq ID 185; CGCTCTTCCGATCT) on the 5' end and the k'-box of the initial reverse primers had a sequence element kₐ' (Seq ID 186; TGCTCTTCCGATCT) on the 5' end (See Fig. 3 for the overview of the sequence tract names).

As listed in Table 7 the k-box of the initial forward primers harbored different k₁ and k₂ sequences and the k'-box of the initial reverse primers harbored different k₁' and k₂' sequences.

**Table 7: k-box and k'-box element sequences are listed as present in 5'-3'orientation of the forward or reverse primers.**

| **k-box name** | **k₁sequence** | **k₂sequence** | **k**'-**box name** | **k₁'sequence** | k₂' **sequence** |
|---|---|---|---|---|---|
| 1bpV1 | G | G | 1bJ1 | C | C |
| 1bpV2 | A | C | 1bpJ2 | T | G |
| 2bpV1 | AC | G | 2bpJ1 | TG | C |
| 2bpV2 | CA | C | 2bpJ2 | GT | G |
| 3bpV1 | ACC | G | 3bpJ1 | TGG | C |
| 3bpV2 | CAG | C | 3bpJ2 | GTC | G |

First amplification steps were performed in a final volume of 50 µl with final concentrations of 1 x PCR Buffer containing 3 mM MgCl₂, 0.2 mM of each dNTP, 1.0 µM forward primer and 1.0 µM reverse primer and 1 unit AmpliTaq Gold DNA Polymerase (Applied Biosystems, Foster City, CA, USA) and the following cycle conditions: 1 cycle at 95°C for 15 min, 34 cycles at 95°C for 30 s, 65 °C for 45 s and 72°C for 45 sec respectively, and a final 10 min elongation step at 72°C. Primary PCR products were purified using the QIAquick PCR Purification Kit (Qiagen, Hilden, Germany) according to the manufacturer's instructions. DNA concentration was determined via the Qubit® 1.0 Fluorometer (Invitrogen, Darmstadt, Germany). As template for the second amplification 500 pg from the purified first amplification product was used.

For second amplification a pair of adaptor PCR primers comprising a left adaptor PCR primer having a sequence 3_{L}-3_{P}-3_{K} and a right adaptor PCR primer having a sequence a_{L}'₋ 3_{P}'-a_{K}' was employed.

The left adaptor primers had the sequence element a_{L}-a_{P} (Seq ID 187; AATGATACGGCGACCACCGAGATCTACACTCTTTCCCTACACGACGCTCTTCCGATCT).

5' 3' whereas the right adaptor primers had the sequence element 3_{L} - a_{P}' (Seq ID 188 ; CAAGCAGAAGACGGCATACGAGATGTGACTGGAGTTCAGACGTGTGCTCTTCCGATCT).

Furthermore, the k-box of the forward adaptor primer harbored different k₁ sequences and the k'-box of the reverse adaptor primers harbored different k₁' sequences as listed in Table 7.

Since K₁ mismatches of the second amplification primer can be removed at the 3' end by the 3'-5' exonuclease-activity of a proofreading polymerase during the second amplification, the strength of a protective effect of phosphorothioates at (i) the first, (ii) the first and second (iii) and at the first to third position from the 3' end of the k-box and k'-box from the left (forward) and right (reverse) second amplification primer, respectively, was analysed in comparison to primers without protective phosphorothioate bonds.

The second amplification steps were performed (i) with a proofreading polymerase (Phusion High-Fidelity DNA Polymerase (Finnzymes, Espoo, Finland)) or (ii) a polymerase without proofreading activity (AmpliTaq Gold).

For PCRs with proofreading polymerase the second amplification step was performed in a final volume of 50 µl including final concentrations of 1 x Phusion HF Buffer with 1.5 mmol/l MgCl₂, 0.05 mmol/l of each dNTP, 1.0 µmol/l forward primer, 1.0 µmol/l reverse primer and 1 unit Phusion High-Fidelity DNA Polymerase. The following thermal cycling conditions were used for the second amplification: 1 cycle at 98°C for 30 s, 12 cycles at 98°C for 10 s, 58°C for 30 s and 72°C for 30s respectively, and a final 5 min elongation at 72°C.

For PCRs with AmpliTaq-Gold the second amplification step was performed in a final volume of 50 µl with final concentrations of 1 x PCR Buffer, 3 mM MgCl₂, 0.2 mmol/l of each dNTP, 1.0 µmol/l forward primer and 1.0 µM reverse primer and 1 unit AmpliTaq Gold DNA Polymerase. The following thermal cycling conditions were used for the second amplification: 1 cycle at 95°C for 15 min, 23 cycles at 98°C for 10 s, 54 °C for 30 s and 72°C for 30 s, respectively, and a final 5 min elongation step at 72°C.

PCR products were analysed on a 6% acrylamide gels and Tif files were produced with Biorad Geldoc 2000 (München, Germany) using default conditions. PCR bands were further quantified with the FusionCapt Advance software (Vilber Lourmat, Eberhardzell, Germany). For quantification of the PCR products from each gel equal areas in size (= gates) were analysed from (i) a gel quantification standard (= a Peer PCR product, 8 µl) which was set to 100% for each analysis, (ii) the PCR products (iii) a no template control (NTC) and (iv) the background gate. As FusionCapt Advance software parameters linear background subtraction was set for each gel in the middle of the background band and a rolling ball background subtraction (size = 11) was employed. With the help of Microsoft Excel the mean and standard derivation (SDN) of replicated experiments was determined.

### Results

An overview of the initial results regarding the K₁-mediated suppression of contamination by mismatches in the K-boxes including the impact of the number of phosphorothioate bonds is given in table 8. The second amplification primers in these experiments had 0-3 phosphorothioate bonds.

In this experiment a 100% "contamination was simulated (A PCR product amplified with the Peer specific first amplification primers described above, employing Peer DNA as template) by adding a PCR product generated in a first PCR round into the second PCR. The second PCR was performed with matching second amplification primers (K₁ = 0 bp mismatch) and second amplification primers with 1 bp and 2 bp K₁ mismatches (summing up the K₁ mismatches of the forward and reverse primer).

The usage of mismatched K-boxes leads to a strong reduction of amplification which was more pronounced when using 2 bases as compared to only one base. This demonstrates the correctness of our concept.

The single results of the experiments are listed in table 8. A comprehensive summary statistic with mean and standard deviation of the results in table 8 is given further below in table 9.

**Table 8: Effect of the number of K₁ mismatches (1 and 2 bp) on contamination suppression employing second amplification primers with 3, 2, 1 and 0 phosphorothioate (PT) bonds at their 3'end and proofreading polymerase. As gel quantification standard the same Peer TCR PCR product was used on each gel for normalization of the PCR product quantities and was set to 100%. Therefore, Vol % larger than 100% can be achieved. Relevant for the effect of contamination suppression are the summary statistics (table 9). (FA = first amplification, SA = second amplification, Gel quant (quantification) standard = the same Peer TCR PCR product was used on each gel as standard for normalization and was set to 100%, NTC no template control, bp = base pair, Vol. % volume percent as determined by FusionCapt Advance software, PT = phosphorothioate bond).**

| **Analysed sample / FA primer combination** | **SA primer combination** | **K₁ mismatch (bp)** | **Vol. % (3 PT)** | **Vol. % (2 PT)** | **Vol. % ( 1 PT)** | **Vol. % (0 PT)** |
|---|---|---|---|---|---|---|
| Gel quant standard | | | 100.0 | 100.0 | 100.0 | 100.0 |
| Peer 1bpV1 1bpJ1 | 1bpV1 1bpJ1 | 0 | 136.7 | 163.1 | 152.4 | 176.9 |
| Peer 1bpV1 1bpJ1 | 1bpV1 1bpJ2 | 1 | 28.7 | 38.4 | 63.6 | 171.8 |
| Peer 1bpV1 1bpJ1 | 1bpV2 1bpJ1 | 1 | 41.6 | 40.9 | 69.6 | 169.9 |
| Peer 1bpV1 1bpJ1 | 1bpV2 1bpJ2 | 2 | 17.0 | 12.2 | 28.7 | 127.7 |
| NTC 1bpV1 1bpJ1 | 1bpV1 1bpJ1 | 0 | 13.7 | 10.8 | 11.6 | 14.0 |
| Background | | | 12.0 | 9.7 | 8.8 | 15.7 |
| Gel quant standard | | | 100.0 | 100.0 | 100.0 | 100.0 |
| Peer 1bpV1 1bpJ2 | 1bpV1 1bpJ1 | 1 | 8.9 | 20.1 | 18.6 | 109.3 |
| Peer 1bpV1 1bpJ2 | 1bpV1 1bpJ2 | 0 | 84.8 | 119.5 | 100.5 | 122.0 |
| Peer 1bpV1 1bpJ2 | 1bpV2 1bpJ1 | 2 | 7.8 | 12.7 | 10.8 | 76.7 |
| Peer 1bpV1 1bpJ2 | 1bpV2 1bpJ1 | 1 | 13.9 | 22.0 | 25.8 | 104.3 |
| NTC 1bpV1 1bpJ2 | 1bpV1 1bpJ1 | 1 | 9.5 | 12.0 | 7.5 | 9.6 |
| Background | | | 7.7 | 11.0 | 8.0 | 7.9 |
| Gel quant standard | | | 100.0 | 100.0 | 100.0 | 100.0 |
| Peer 1bpV2 1bpJ1 | 1bpV1 1bpJ1 | 1 | 19.3 | 28.2 | 45.1 | 87.0 |
| Peer 1bpV2 1bpJ1 | 1bpV1 1bpJ2 | 2 | 10.6 | 11.9 | 14.2 | 80.5 |
| Peer 1bpV2 1bpJ1 | 1bpV2 1bpJ1 | 0 | 82.4 | 83.9 | 86.8 | 101.3 |
| Peer 1bpV2 1bpJ1 | 1bpV2 1bpJ2 | 1 | 11.7 | 16.8 | 21.3 | 78.0 |
| NTC 1bpV2 1bpJ1 | 1bpV1 1bpJ1 | 1 | 9.2 | 8.5 | 8.9 | 7.8 |
| Background | | | 7.0 | 12.0 | 6.8 | 8.2 |
| Gel quant standard | | | 100.0 | 100.0 | 100.0 | 100.0 |
| Peer 1bpV2 1bpJ2 | 1bpV1 1bpJ1 | 2 | 10.1 | 10.2 | 11.8 | 81.7 |
| Peer 1bpV2 1bpJ2 | 1bpV1 1bpJ2 | 1 | 22.7 | 22.5 | 38.2 | 113.8 |
| Peer 1bpV2 1bpJ2 | 1bpV2 1bpJ1 | 1 | 11.4 | 11.3 | 13.1 | 103.0 |
| Peer 1bpV2 1bpJ2 | 1bpV2 1bpJ2 | 0 | 94.0 | 81.1 | 78.7 | 96.9 |
| NTC 1bpV2 1bpJ2 | 1bpV1 1bpJ1 | 2 | 9.3 | 8.2 | 8.1 | 10.0 |
| Background | | | 9.7 | 7.9 | 8.2 | 10.2 |

**Table 9: Summary statistics of table 8 (bp = base pair, Vol % volume percent, SDN = standard deviation, PT = phosphorothioate bond). Quantities around 100% Vol. means that there is no suppression of contamination. A lower Vol. % is the result of contamination suppression.**

| **K₁ mismatch (bp)** | **Vol. % (3 PT) Mean** | **Vol. % (3 PT) SDN** | **Vol. % (2 PT) Mean** | **Vol. % (2 PT) SDN** | **Vol. % (1 PT) Mean** | **Vol. % (1 PT) SDN** | **Vol. % (0 PT) Mean** | **Vol. % (0 PT) SDN** |
|---|---|---|---|---|---|---|---|---|
| **0** | 99.5 | 21.9 | 111.9 | 33.2 | 104.6 | 28.7 | 124.3 | 31.8 |
| **1** | 19.8 | 10.3 | 25.0 | 9.6 | 36.9 | 19.7 | 117.1 | 32.9 |
| **2** | 11.4 | 3.4 | 11.8 | 0.9 | 16.4 | 7.2 | 91.7 | 20.9 |

Taken together, table 8 and 9 demonstrate that in a setting with a proofreading polymerase employed in the second amplification, contamination suppression is much more effective with phosphorothioate bonds at the 3'end of the k-box and k'-box of reamplification primers. Furthermore, an increasing number of K₁ mismatches leads to improved contamination suppression. For example with 2 bp K₁ mismatches and 2 phophorothioate bonds the contamination (mean of 11.8, SDN: 0.9) is almost suppressed to NTC or background level. The mean of all NTCs in table 8 was 9.7 (SDN: 1.9) and the mean of background was 9.4 (SDN: 2.3). Further experiments with longer K₁ mismatches (see below) revealed a complete suppression of contamination.

In a further experiment the effectiveness of K₁ mismatches of 2, 3, 4, and 6 bp total length to suppress contaminations was analysed in comparison to controls without K₁ mismatches. The second amplification primers in these experiments had 3 phosphorothioate bonds. The results are given in table 10. A summary statistic with mean and standard deviation of the results in table 10 is given in table 11.

**Table 10 Effect of the number of K₁ mismatches (2, 3 4 and 6 bp total length) on contamination suppression employing second amplification primers with 3 phosphorothioate bonds at their 3'end and proofreading polymerase. Quantities around 100% Vol. means that there is no contaminations suppression. A lower Vol. % is a result of contamination suppression. (FA = first amplification, SA = reamplification, Gel quant (quantification) standard = the same Peer TCR PCR product was used on each gel as standard for normalization of PCR product quantity and was set to 100%, NTC = no template control, bp = base pair, Vol. % volume percent, PT = phosphorothioate bond).**

| **Analysed sample / FA primer combination** | **SA primer combination** | **Vol. % (3 PT)** | **K₁ mismatch (bp)** |
|---|---|---|---|
| Gel quant standard | | 100.0 | |
| Peer 2bpV1 2bpJ1 | 2bpV1 2bpJ1 | 113.8 | 0 |
| Peer 2bpV1 2bpJ1 | 2bpV1 2bpJ2 | 11.5 | 2 |
| Peer 2bpV1 2bpJ1 | 2bpV2 2bpJ1 | 10.4 | 2 |
| Peer 2bpV1 2bpJ1 | 2bpV2 2bpJ2 | 10.9 | 4 |
| NTC 2bpV1 2bpJ1 | 2bpV1 2bpJ1 | 10.6 | 0 |
| Background | | 9.5 | |
| Gel quant standard | | 100.0 | |
| Peer 2bpV1 2bpJ2 | 2bpV1 2bpJ1 | 9.8 | 2 |
| Peer 2bpV1 2bpJ2 | 2bpV1 2bpJ2 | 99.3 | 0 |
| Peer 2bpV1 2bpJ2 | 2bpV2 2bpJ1 | 8.5 | 4 |
| Peer 2bpV1 2bpJ2 | 2bpV2 2bpJ2 | 8.9 | 2 |
| NTC 2bpV1 2bpJ2 | 2bpV1 2bpJ1 | 7.6 | 2 |
| Background | | 8.0 | |
| Gel quant standard | | 100.0 | |
| Peer 2bpV2 2bpJ1 | 2bpV1 2bpJ1 | 11.2 | 2 |
| Peer 2bpV2 2bpJ1 | 2bpV1 2bpJ2 | 8.1 | 4 |
| Peer 2bpV2 2bpJ1 | 2bpV2 2bpJ1 | 88.9 | 0 |
| Peer 2bpV2 2bpJ1 | 2bpV2 2bpJ2 | 8.5 | 2 |
| NTC 2bpV2 2bpJ1 | 2bpV1 2bpJ1 | 7.7 | 2 |
| Background | | 9.4 | |
| Gel quant standard | | 100.0 | |
| Peer 2bpV2 2bpJ2 | 2bpV1 2bpJ1 | 10.8 | 4 |
| Peer 2bpV2 2bpJ2 | 2bpV1 2bpJ2 | 13.1 | 2 |
| Peer 2bpV2 2bpJ2 | 2bpV2 2bpJ1 | 9.6 | 2 |
| Peer 2bpV2 2bpJ2 | 2bpV2 2bpJ2 | 98.5 | 0 |
| NTC 2bpV2 2bpJ2 | 2bpV1 2bpJ1 | 11.8 | 4 |
| Background | | 11.1 | |
| Gel quant standard | | 100.0 | |
| Peer 3bpV1 3bpJ1 | 3bpV1 3bpJ1 | 70.1 | 0 |
| Peer 3bpV1 3bpJ1 | 3bpV1 3bpJ2 | 12.1 | 3 |
| Peer 3bpV1 3bpJ1 | 3bpV2 3bpJ1 | 22.4 | 3 |
| Peer 3bpV1 3bpJ1 | 3bpV2 3bpJ2 | 12.8 | 6 |
| NTC 3bpV1 3bpJ1 | 3bpV1 3bPJ1 | 19.0 | 0 |
| Background | | 9.9 | |
| Gel quant standard | | 100.0 | |
| Peer 3bpV1 3bpJ2 | 3bpV1 3bpJ1 | 10.0 | 3 |
| Peer 3bpV1 3bpJ2 | 3bpV1 3bpJ2 | 79.8 | 0 |
| Peer 3bpV1 3bpJ2 | 3bpV2 3bpJ1 | 9.2 | 6 |
| Peer 3bpV1 3bpJ2 | 3bpV2 3bpJ2 | 17.5 | 3 |
| NTC 3bpV1 3bpJ2 | 3bpV1 3bpJ1 | 8.4 | 3 |
| Background | | 8.2 | |
| Gel quant standard | | 100.0 | |
| Peer 3bpV2 3bpJ1 | 3bpV1 3bpJ1 | 8.6 | 3 |
| Peer 3bpV2 3bpJ1 | 3bpV1 3bpJ2 | 7.0 | 6 |
| Peer 3bpV2 3bpJ1 | 3bpV2 3bpJ1 | 71.5 | 0 |
| Peer 3bpV2 3bpJ1 | 3bpV2 3bpJ2 | 7.6 | 3 |
| NTC 3bpV2 3bpJ1 | 3bpV1 3bpJ1 | 6.8 | 3 |
| Background | | 7.8 | |
| Gel quant standard | | 100.0 | |
| Peer 3bpV2 3bpJ2 | 3bpV1 3bpJ1 | 7.2 | 6 |
| Peer 3bpV2 3bpJ2 | 3bpV1 3bpJ2 | 7.4 | 3 |
| Peer 3bpV2 3bpJ2 | 3bpV2 3bpJ1 | 9.2 | 3 |
| Peer 3bpV2 3bpJ2 | 3bpV2 3bpJ2 | 61.4 | 0 |
| NTC 3bpV2 3bpJ2 | 3bpV1 3bpJ1 | 8.0 | 6 |
| Background | | 8.8 | |

**Table 11: Summary statistics of table 10. Quantities around 100% Vol. means that there is no contaminations suppression. A lower Vol. % is a result of the contamination suppression. (bp = base pair, Vol. % volume percent, SDN = standard deviation).**

| **K₁ mismatch** | **Vol. % Mean** | **Vol. % SDN** |
|---|---|---|
| 0 | 85.4 | 16.7 |
| 2 | 10.4 | 1.4 |
| 3 | 11.9 | 5.0 |
| 4 | 9.6 | 1.3 |
| 6 | 9.1 | 2.3 |

In table 10 the mean of all NTCs was 10.0 (SDN: 3.7) and the mean of background was 9.1 (SDN: 1.0).

In summary, table 10 and 11 show that in a setting with a proofreading polymerase employed in second amplification and 3 phosphorothioate bonds at the 3'end of the k-box and k'-box of the second amplification primers K₁ mismatches of 4 bp (mean 9.6, SDN 1.3) and 6 bp (mean 9.1, SDN 2.3) lead to complete suppression of contaminations comparable to NTCs and background.

In another experiment the effect of the number of K₁ mismatches (1 and 2 bp) on contamination suppression employing second amplification primers without phosphorothioate bonds and a polymerase without proofreading activity (AmpliTaq Gold) was analysed (Table 12). Summary statistics for table 12 are provided in table 13.

**Table 12: Effect of the number of K₁ mismatches (1 and 2 bp total length) on contamination suppression employing second amplification primers without phosphorothioate bonds and a polymerase without proofreading activity. Quantities around 100% Vol. means that there is no contaminations suppression. A lower Vol. % is a result of contamination suppression. (AmpliTaq Gold). (FA = first amplification, SA = second amplification, Gel quant (quantification) standard = the same Peer TCR PCR product was used on each gel as standard for normalization of PCR product quantity and was set to 100%I, bp = base pair, Vol. % volume percent)**

| **Analysed sample /FA primer combination** | **SA primer combination** | **Vol. %** | **K₁ mismatch** |
|---|---|---|---|
| Gel quant standard | - | 100 | |
| Peer 1bpV1 1bpJ1 | 1bpV1 1bpJ1 | 155.7 | 0 |
| Peer 1bpV1 1bpJ1 | 1bpV2 1bpJ1 | 92.1 | 1 |
| Peer 1bpV1 1bpJ1 | 1bpV2 1bpJ2 | 54.9 | 2 |
| NTC 1bpV1 1bpJ1 | 1bpV1 1bpJ1 | 13.8 | 0 |
| NTC 1bpV1 1bpJ1 | 1bpV2 1bpJ1 | 14.2 | 1 |
| NTC1bpV1 1bpJ1 | 1bpV2 1bpJ2 | 11.9 | 2 |
| Background | - | 13.2 | |
| Gel quant standard | - | 100 | |
| Peer 1bpV1 1bpJ2 | 1bpV2 1bpJ1 | 51.0 | 2 |
| Peer 1bpV1 1bpJ2 | 1bpV1 1bpJ2 | 115.0 | 0 |
| Peer 1bpV1 1bpJ2 | 1bpV2 1bpJ2 | 84.7 | 1 |
| NTC 1bpV1 1bpJ2 | 1bpV2 1bpJ1 | 10.3 | 2 |
| NTC 1bpV1 1bpJ2 | 1bpV1 1bpJ2 | 10.8 | 0 |
| NTC 1bpV1 1bpJ2 | 1bpV2 1bpJ2 | 9.4 | 1 |
| Background | - | 9.8 | |
| Gel quant standard | - | 100 | |
| Peer 1bpV2 1bpJ1 | 1bpV1 1bpJ1 | 104.6 | 1 |
| Peer 1bpV2 1bpJ1 | 1bpV2 1bpJ1 | 120.3 | 0 |
| Peer 1bpV2 1bpJ1 | 1bpV1 1bpJ2 | 77.8 | 2 |
| NTC 1bpV2 1bpJ1 | 1bpV1 1bpJ1 | 38.0 | 1 |
| NTC 1bpV2 1bpJ1 | 1bpV2 1bpJ1 | 30.2 | 0 |
| NTC 1bpV2 1bpJ1 | 1bpV1 1bpJ2 | 20.3 | 2 |
| Background | - | 14.5 | |
| Gel quant standard | - | 100 | |
| Peer 1bpV2 1bpJ2 | 1bpV1 1bpJ1 | 50.1 | 2 |
| Peer 1bpV2 1bpJ2 | 1bpV2 1bpJ1 | 62.7 | 1 |
| Peer 1bpV2 1bpJ2 | 1bpV2 1bpJ2 | 89.1 | 0 |
| NTC 1bpV2 1bpJ2 | 1bpV1 1bpJ1 | 12.5 | 2 |
| NTC 1bpV2 1bpJ2 | 1bpV2 1bpJ1 | 16.5 | 1 |
| NTC 1bpV2 1bpJ2 | 1bpV2 1bpJ2 | 15.0 | 0 |
| Background | - | 11.2 | |

**Table 13: A summary statistics of table 12 is provided. Quantities around 100% Vol. means that there is no contaminations suppression. A lower Vol. % is a result of contamination suppression. (bp = base pair, Vol. % volume percent, SDN = standard deviation).**

| **K₁ mismatches** | **Vol. % Mean** | **Vol. % SDN** |
|---|---|---|
| 0 | 120.0 | 23.7 |
| 1 | 86.0 | 15.2 |
| 2 | 58.5 | 11.3 |

The mean of all NTCs in table 12 was 16.9 (SDN: 8.0) and the mean of background was 12.2 (SDN: 1.8).

In summary, table 12 and 13 show that also in a setting with a polymerase without proofreading activity and second amplification primers without phosphorothioate bonds at the 3'end of the k-box and k'-box contamination suppression increases with an increasing number of K₁ mismatches.

### GeneScan analysis of contamination suppression by K₁.

GeneScan analysis allows a quantitative analysis of PCR products with a size resolution of 1 bp difference. Thereby the right PCR second amplification primer is fluourescence labeled and therefore fluorescence intensities of the single PCR products can be quantified and analyzed. In other words different TCR length can be quantitatively analyzed such as the TCR from the Peer cell line with a PCR product length of 252 bp in comparison to the intensities of PCR products (TCRs) with a different length. Further details are described below.

Employing GeneScan analysis, a proof of principle experiment was performed to demonstrate that K₁ can suppress contaminations when templates are analysed which harbor many different TCR sequences. Tonsillar DNA - with different T-cells and therefore TCR gene rearrangements of different length - as template was analysed together with spiked in DNA from the T-cell line Peer harboring only one TCR.

To illustrate the overall experimental design, a Peer amplicon from a "Monday amplification" with a specific Mondays K₁ element was mixed as contamination simulation in a Tuesday mixture of tonsillar TCR amplicons with a Tuesdays K₁ element. GeneScan analysis which makes a quantification of the TCRs of different length possible was used as readout to demonstrate that the Peer contamination was suppressed already by a Monday/Tuesday k₁ mismatch of only 2 bp in comparison to control reactions without K₁ mismatch.

For these experiments, three different primary templates were prepared: (A) tonsillar DNA (100%), (B) Peer DNA (100%) and (C) a mix of tonsillar DNA (80%) with Peer DNA (20 %). A primary PCR was performed as described above with one modification: Template (A) and (C) was amplified with 40 PCR cycles instead of 34 cycles to obtain a sufficient amount of the TCR PCR product since the monoclonal Peer TCR was amplified more easily than the polyclonal TCRs in the tonsillar DNA.

The first amplification primers described above were used with the k-boxes "1bpV1", "1 bpV2" and the k'-boxes "1bpJ1" and "1bpJ2" (Table 14).

To simulate contamination and to demonstrate suppression of contamination 80% of a tonsillar primary PCR product (comprising different TCRs) was mixed with 20% of a Peer primary amplicon (Table 14, number 1-4). Thereby the Peer primary amplicon had a 2 bp K₁ mismatch to the tonsillar amplicons (Table 14, number 1-4). Thereafter, the second PCR was amplified with second amplification primers that had only a K₁ match with the tonsillar amplicons but not with the "contamination simulating" Peer amplicon. As a control, the PCR product obtained from template "C" (tonsillar DNA 80% with Peer DNA 20 %) without K₁ mismatch was used (Table 14, number 5-8), which was amplified with matching K₁ second amplification primers. Furthermore, for all primer combinations no template controls (NTC) were performed.

Second amplification primers were used as described above with K-boxes listed in Table 14. The K-boxes of all secondary primers had two phosphorothioate bonds at their 3'end. The right second amplification primers were fluorescence (FAM) labelled to allow GeneScan quantification (3130 Genetic Analyser, Applied Biosystems) and analysis with GeneMapper software 4.0 (Applied Biosystems) of PCR products.

**Table 14: Primer combinations employed in experiments to demonstrate contamination suppression by GeneScan analysis (Nr. = Number).**

| **Nr.** | **First amplification** | | **Second amplification** | **K₁ mismatch** | **Function** |
|---|---|---|---|---|---|
| | **Peer** | **Tonsil** | **Peer-Tons-Mix** | | |
| 1 | 1bpV1 1bpJ1 | <-------- 1bpV21bpJ2 --------> | | 2bp | contamination suppression |
| 2 | 1bpV1 1bpJ2 | <-------- 1bpV2 1bpJ1 --------> | | 2bp | contamination suppression |
| 3 | 1bpV2 1bpJ1 | <-------- 1bpV1 1bpJ2 --------> | | 2bp | contamination suppression |
| 4 | 1bpV2 1bpJ2 | <-------- 1bpV1 1bpJ1 --------> | | 2bp | contamination suppression |
| 5 | <-------- 1bpV1 1bpJ1 --------> | | | 0 bp | control |
| 6 | <-------- 1bpV1 1bpJ2 --------> | | | 0 bp | control |
| 7 | <-------- 1bpV2 1bpJ1 --------> | | | 0 bp | control |
| 8 | <-------- 1bpV2 1bpJ2 --------> | | | 0 bp | control |

### Results:

In table 15 and 16, the results of GeneScan analysis are provided with the experiment numbers used in table 14, and all peaks (= PCR products) detected listed according to their length (in bp) with their peak height (= fluorescence intensity) and peak area. Peaks below a threshold height of 50 are not shown. All NTC peaks were below this threshold. All experiments were performed in duplicates (not shown) with similar results.

Taken together the results demonstrate that the Peer TCR PCR product with a length of 252 bp, is suppressed in the "contamination simulation" experiments (table 15). In the control experiments (table 16) the Peer TCR product is the dominant peak. In conclusion, the Peer contamination was suppressed already by a K₁ mismatch of only 2 bp in comparison to control reactions without K₁ mismatch.

**Table 15: Experiment number for contamination suppression with the primer combination listed in table 14, and all GeneScan peaks detected according to their size (= length in bp) with their height and area are shown. The most prominent peak for each experiment is underlined, which are all different from the Peer TCR PCR amplicon size of 252 bp (Peaks threshold height > 50).**

| **Number** | **Size** | **Height** | **Area** |
|---|---|---|---|
| 1 | 237.83 | 72 | 655 |
| 1 | 238.94 | 130 | 1019 |
| 1 | 241.53 | 50 | 410 |
| 1 | 242.64 | 219 | 1634 |
| 1 | **243.66** | **413** | **2994** |
| 1 | 244.58 | 87 | 645 |
| 1 | 245.5 | 79 | 700 |
| 1 | 246.61 | 129 | 1162 |
| 1 | 249.74 | 194 | 3536 |
| 1 | 251.03 | 169 | 1636 |
| 1 | 252.04 | 304 | 3887 |
| 1 | 253.42 | 205 | 1575 |
| 1 | 254.25 | 188 | 1407 |
| 1 | 255.26 | 291 | 2006 |
| 2 | 237.7 | 97 | 677 |
| 2 | 238.72 | 167 | 1293 |
| 2 | 239.73 | 80 | 784 |
| 2 | 243.98 | 55 | 902 |
| 2 | 245.0 | 205 | 1417 |
| 2 | **246.01** | **372** | **2637** |
| 2 | 248.41 | 59 | 609 |
| 2 | 249.51 | 99 | 1100 |
| 2 | 250.99 | 116 | 868 |
| 2 | 252.09 | 195 | 1740 |
| 2 | 253.84 | 211 | 1550 |
| 2 | 254.85 | 385 | 2571 |
| 2 | 269.04 | 51 | 343 |
| 3 | 237.7 | 152 | 1312 |
| 3 | **238.82** | **308** | **2579** |
| 3 | 240.03 | 155 | 1377 |
| 3 | 242.54 | 80 | 645 |
| 3 | 243.56 | 149 | 1166 |
| 3 | 245.14 | 79 | 902 |
| 3 | 246.16 | 116 | 916 |
| 3 | 248.01 | 87 | 685 |
| 3 | 249.03 | 153 | 1115 |
| 3 | 249.95 | 61 | 425 |
| 3 | 251.06 | 126 | 956 |
| 3 | 252.17 | 200 | 1477 |
| 3 | 254.11 | 60 | 408 |
| 3 | 255.04 | 96 | 716 |
| 4 | 237.55 | 156 | 1261 |
| 4 | 238.57 | 258 | 1977 |
| 4 | 241.17 | 120 | 907 |
| 4 | 242.19 | 274 | 1991 |
| 4 | 243.21 | 225 | 1789 |
| 4 | 245.25 | 63 | 487 |
| 4 | 246.27 | 108 | 861 |
| 4 | 247.2 | 67 | 455 |
| 4 | 248.12 | 214 | 1823 |
| 4 | **249.23** | **385** | **2974** |
| 4 | 250.25 | 134 | 1053 |
| 4 | 251.18 | 108 | 789 |
| 4 | 252.19 | 174 | 1315 |
| 4 | 265.91 | 77 | 577 |

**Table 16: Experiment number for controls listed in table 14, and all GeneScan peaks detected according to their size (length in bp) with their height and area are shown. The most prominent peak for each experiment is underlined, which are all 252 bp long, the size of the Peer TCR PCR amplicon (Peaks threshold height > 50).**

| **Number** | **Size** | **Height** | **Area** |
|---|---|---|---|
| 5 | 246.03 | 64 | 580 |
| 5 | 247.04 | 53 | 510 |
| 5 | 248.04 | 57 | 491 |
| 5 | 249.14 | 120 | 1079 |
| 5 | 250.05 | 318 | 2766 |
| 5 | 251.06 | 1215 | 9882 |
| 5 | **252.06** | **1835** | **14840** |
| 6 | 246.1 | 58 | 438 |
| 6 | 249.06 | 82 | 685 |
| 6 | 250.08 | 228 | 1838 |
| 6 | 251.1 | 1052 | 7726 |
| 6 | **252.11** | **1906** | **13148** |
| 6 | 253.13 | 300 | 2336 |
| 7 | 246.17 | 73 | 600 |
| 7 | 247.18 | 56 | 491 |
| 7 | 249.2 | 145 | 1370 |
| 7 | 250.21 | 293 | 2353 |
| 7 | 251.22 | 1078 | 8489 |
| 7 | **252.23** | **1902** | **14873** |
| 8 | 245.03 | 51 | 411 |
| 8 | 246.15 | 85 | 733 |
| 8 | 247.26 | 66 | 523 |
| 8 | 248.28 | 75 | 562 |
| 8 | 249.12 | 158 | 1345 |
| 8 | 250.23 | 303 | 2468 |
| 8 | 251.16 | 1260 | 9763 |
| 8 | **252.27** | **2383** | **16695** |
| 8 | 253.2 | 375 | 3249 |

### Example 2: Proof of principle for k₂ and k₂' function:

As a short sequence element, k₂ is located at the 3'of the sequence element k₁ and k₂' is a sequence element located at the 3'-end of the sequence element k₁' (Fig. 2-3). K₂ serve to individualize the first primer pair of the set and have no complementary sequence elements on the second ("adaptor") primers. K₂ sequences are designed to detect contamination from previous amplification reactions and therefore control the suppression efficiency of K₁.

It is assumed that each day of the week one primer set is used comprising one for the day specific k₂ and/or k₂' sequence in the first amplification primer as well as a k₁ and k₁' sequence matching the first and second amplification primer. Then a contamination can be clearly identified by a mismatched k₂ (or k₂') element if a Tuesday second amplification product contains k₂/k₂' elements of the Monday amplification product but k₁ and k₁' sequences of the Tuesday second amplification primers. In this case the contamination is caused by nonspecific priming of Tuesday k₁ and k₁' element of the second amplification primers to the Monday k₁ and k₁' element in the first amplification product. Furthermore the contamination amplification could be caused by partial or full degradation of the Tueseday k₁ and k₁' element by polymerases with proofreading activity. Since the k₂/k₂' elements are only present in the first amplification primers the contamination can still be identified in the second amplification product. Therefore, k₂/k₂' elements can be seen as a valuable safe lock mechanism to detect contaminations, complementing the already significant contribution of k₁/k₁' sequences to avoid such contaminations. There is a synergistic control function of k₂/k₂' that ensures the k₁/k₁' contamination suppression works 100%.

In order to demonstrate the function and effectiveness of k₂/k₂' sequence tracts to detect contaminations a first and second amplification with primers specific for the Peer TCR as described above were used with the following k-box and k'-box elements for the first forward amplification primer given in table 7:
Monday first amplification: forward primer 1 bpV1 and reverse primer 1bpJ1
Tuesday second amplification: forward primer 1 bpV2 and reverse primer 1bpJ1

Therefore, there is 1 bp k₁ mismatch between the Monday first amplification primer (k₁ = "G") and the Tuesday second amplification primer (k₁ = "A"). Furthermore, the Monday first amplification primer had the k₂ = "G" (Table 7).

The first Monday amplification was regarded to be a "100 % contamination" (Monday primary amplification product) of Tuesday second amplification. Therefore, a second amplification was performed with Tuesday second amplification primers and the Monday first amplification product as template. In the gel analysis of the resulting second amplification PCR product there was a PCR product detectable, since due to the only 1 bp long k₁ mismatch this Monday contamination was not completely suppressed during second amplification (with Tuesday second amplification primers). This PCR product was sequenced.

Sanger sequencing of the amplicon identified the k₂ sequence of the amplicon as identifier of the Monday primary amplification forward primer (k₂ = "G"). Therefore, in this case the Monday specific k₂' sequence (k₂ = "G") identified the contamination from the Monday primary amplification product in the Tuesday second amplification (The Tuesday k₂ sequence would have been "C" for the Tuesday k-box 1 bpV2 Table 7).

To gain a deeper understanding of this contamination detection and prevention system the second amplification in this experiment was performed independently with proofreading polymerase and with a polymerase without proofreading activity, with the PCR conditions described above for these reactions. As a result, in both experiments the contamination (Monday sample) could be identified by Sanger sequencing due to the contamination specific k₂ sequence (k₂ = "G").

The sequencing results revealed that with proofreading polymerase the k₁ sequence from the contaminating (Monday) sample was found, whereas in the second amplification employing a polymerase without proofreading activity k₁ sequences from the second amplification primers (Tuesday amplification) were present. This is due to fact that there was a k₁ mismatch between the first and second amplification reverse primer and the k₁ element of the second amplification primer was removed (degraded) at the 3' end by the 3' - 5' exonuclease-activity of a proofreading polymerase during the second amplification, despite the second amplification primers harboring two phosphorothioate bonds. In contrast, the k₁ element of the second amplification primer was not removed using a polymerase without proofreading activity.

Taken together, the Sanger sequencing demonstrated the k₂/k₂' function to detect contamination. Thereby polymerase with or without proofreading polymerase can be used in second amplification. Importantly k₂/k₂' elements help to understand and control the function of k₁/k₁'.

### 3) Proof of principle for S function

A feature that improves on the performance of the above elements k₁ (and k₁') and k₂ (and k₂') is the introduction of short separator sequences S and S' (Fig. 3-4). S separates the constant initial primer sequence p_{c} from the sequence tracts k₁ and k₂ and s' separates the constant initial primer sequence p_{c}' from k₁' and k₂' respectively. Since k₁ /k₁' and k₂/k₂' vary among different primers used in subsequent reactions, it may well be that some variations of k₁/k₁' and / or k₂/k₂' coincidentally match in their last nucleotides on the 3' terminal end the sequence of the target next to the hybridizing part of the initial primer, p_{c} or p_{c}'. Thus, the target sequence-matching tract of the initial primer would be elongated, leading to higher annealing temperatures and thus, possibly, PCR bias.

As a proof of principle that S reduces PCR bias a simulation of an incidentally match of 6 bp length of the k-box and k'-box in the first amplification primers to the target sequence was analysed with S of 1, 2 and 3 bp length and no S sequence for comparison.

The first amplification was performed as described above with 100 ng template DNA from the T-cell lymphoma cell line Peer and the following cycle conditions. 1 cycle at 95°C for 15 min, 29 cycles at 95°C for 30 s, 68 °C for 45 s and 72°C for 45 sec respectively, and a final 10 min elongation at 72°C.

The first amplification PCR primers had a sequence k-p_{c} with the matching sequence p_{c} to the V-4 segment (Seq ID 189; ACCTACACACCCTGC), whereas the right first amplification primers which had the sequence and k'-p_{c}' had the matching sequence p_{c}' (Seq ID 190; AGCCGGGTGCCTGG) to the J-2.1 segment. Furthermore, the k-box of the left first amplification primers had a sequence element kₐ (Seq ID 191; CGCTCTTCCGATCT) on the 5' terminus and the k'-box of the right first amplification primers had a sequence element kₐ' (Seq ID 192 ; TGCTCTTCCGATCT) on the 5' terminus.

An overview of the 6bp matching K-box sequences to the V-4 segment and J-2.1 segment together with the s sequences of different length are given in table 17.

**Table 17: Overview of S sequences and 6 bp K-box sequences. Some of the K-box sequences have a full-match to the V-4 segment and J-2.1 segment to simulate an incidentally matched K-box to the template sequence.**

| **K-box name** | **Primer** | **K-box sequence** | **Template sequence match** | **S sequence** |
|---|---|---|---|---|
| VKM | forward | TCCTTC | Yes | none |
| VKMS1 | forward | TTCCTT | Yes | G |
| VKMS2 | forward | ATTCCT | Yes | GG |
| VKMS3 | forward | TATTCC | Yes | AGG |
| VKMM | forward | CAACGT | No | none |
| VKMMS1 | forward | GGTTCA | No | G |
| VKMMS2 | forward | GGAGTA | No | GG |
| VKMMS3 | forward | GCACTT | No | AGG |
| JKM | reverse | ACTGTC | yes | none |
| JKMS1 | reverse | CACTGT | yes | T |
| JKMS2 | reverse | GCACTG | yes | GT |
| JKMS3 | reverse | AGCACT | yes | CGT |
| JKMM | reverse | TGACGA | No | none |
| JKMMS1 | reverse | GTTGAC | No | T |
| JKMMS2 | reverse | ATGACT | No | GT |
| JKMMS3 | reverse | GTTGAG | No | CGT |

**Table 18: Experiment Nr. and results of proof of principle experiments to show that S can help to avoid a PCR bias by preventing K-box matches to the DNA template and therefore preventing unequal primer annealing temperatures and different amplification rates. Gel quant (quantification) S. (standard) = the same Peer TCR PCR product was used on each gel as standard for normalization of PCR product quantity and was set to 100 %. Vol. % = volume percent, E1-5 = Experimen 1-5 (Replicates), SDN = standard deviation).**

| **Nr** . | **Sample** | **Primer** | **Vol. % E1** | **Vol. % E2** | **Vol. % E3** | **Vol. % E4** | **Vol. % E5** | **Mean** | **SDN** |
|---|---|---|---|---|---|---|---|---|---|
| 1 | Gel quant S. | - | 100 | 100 | 100 | 100 | 100 | 100.0 | 0.0 |
| 2 | Peer | VKM JKM | 67.1 | 56.4 | 54.2 | 48.9 | 65.8 | 58.5 | 7.0 |
| 3 | Peer | VKMM JKMM | 27.6 | 13.6 | 15.1 | 14.9 | 16.7 | 17.6 | 5.1 |
| 4 | Peer | VKMS1 JKMS1 | 30.7 | 24.5 | 27.7 | 22.0 | 29.2 | 26.8 | 3.2 |
| 5 | Peer | VKMS2JKMS2 | 39.8 | 20.5 | 19.5 | 26.6 | 14.9 | 24.3 | 8.6 |
| 6 | Peer | VKMS3JKMS3 | 27.1 | 14.5 | 16.9 | 16.2 | 17.3 | 18.4 | 4.5 |
| 7 | Peer | VKMMS1 JKMMS1 | 16.6 | 11.4 | 14.6 | 13.4 | 13.7 | 13.9 | 1.7 |
| 8 | Peer | VKMMS2 JKMMS2 | 24.2 | 14.3 | 13.6 | 13.4 | 12.9 | 15.7 | 4.3 |
| 9 | Peer | VKMMS3 JKMMS3 | 21.0 | 9.6 | 21.3 | 14.4 | 21.4 | 17.5 | 4.8 |
| 10 | Background | - | 11.3 | 8.5 | 10.9 | 10 | 11.1 | 10.4 | 1.0 |
| | | | | | | | | | |
| 11 | Gel quant S. | - | 100 | 100 | 100 | 100 | 100 | 100.0 | 0.0 |
| 12 | NTC | VKM JKM | 7.2 | 8 | 9.3 | 6.3 | 7.3 | 7.6 | 0.9 |
| 13 | NTC | VKMM JKMM | 9.5 | 7.8 | 9.4 | 6.8 | 6.9 | 8.1 | 1.1 |
| 14 | NTC | VKMS1 JKMS1 | 9.5 | 8.5 | 9.7 | 7.8 | 8.7 | 8.8 | 0.6 |
| 15 | NTC | VKMS2JKMS2 | 9.2 | 9.1 | 10.8 | 6.8 | 7.7 | 8.7 | 1.3 |
| 16 | NTC | VKMS3JKMS3 | 8.6 | 8.2 | 9.2 | 6.7 | 7.1 | 8.0 | 0.8 |
| 17 | NTC | VKMMS1 JKMMS1 | 9.1 | 9.1 | 9.7 | 6.9 | 7.9 | 8.5 | 0.9 |
| 18 | NTC | VKMMS2 JKMMS2 | 7.3 | 7.2 | 9.7 | 7.6 | 7.7 | 7.9 | 0.8 |
| 19 | NTC | VKMMS3 JKMMS3 | 7.7 | 9.1 | 9.5 | 6.7 | 7.7 | 8.1 | 0.9 |
| 20 | Background | - | 8 | 9.1 | 8.6 | 5.8 | 7.5 | 7.8 | 1.0 |

Table 18 shows that S sequences lead to a similar amplification despite of coincidentally template matching k-box and k'-box sequences. For example in table 18 line 6 the amplification (Vol. %) with primers harboring a S sequence of 3 bp length and template matching k-box and k'-box sequences have a mean of 18.4 (SDN 4.5) which is comparable to the amplification without template matching k-box and k'-box sequences in Table 18 line 3 with a mean of 17.6 (SDN 5.1).

This is the proof of the principle that S functions in a synergistic way to avoid PCR bias, due to altered primer annealing temperatures in the case of coincidentally template matching variations of some K-box sequences.

*Design of suitable k₁*/*k₁' and k₂*/*k₂'* sequences.

To provide examples for suitable k₁ and k₁' sequences, they were designed in a way to (a) optimally avoid cross-hybridization between all k₁ and k₁' sequences given in one of the tables 19-21 below, (b) adjust the melting temperatures of k₁ and k₁' sequences in a narrow range of ± 2 degrees Celsius and (c) to avoid low complex base compositions with > 2/3 of the bases being the same nucleotide (A,C,G,T),

Each of the tables 19-21 consists of an equal number of k₁ and k₁' sequences for the forward and reverse primers and within each of the three tables k₁ and k₁' sequences have a specific length (4, 5, or 6 bp).

In detail, features a)-c) were established by comparing all potential k₁ and k₁' sequences of one specific length (4, 5 or 6 bp) against each other and excluded all those which were reverse-complements to any other k₁ and k₁' sequences of this specific length. To further refine the k₁ and k₁' sequences the design algorithm compared in a further step all k₁ and k₁' sequences of one specific length (4, 5, or 6 bp) against all other reverse complement k₁ and k₁' sequences of this specific length and excluded all k₁ and k₁' sequences which either had >2 common bases at the 3' terminal end of the k₁ and k₁' sequences or had >60% bases in common with another k₁ and k₁' sequence.

The final results of this optimized k₁ and k₁' sequences are given in tables 19-21. It is understood that this are examples and that other optimized k₁-boxes with different selection criteria are possible.

Furthermore, examples of suitable k₂ /k₂' sequences are provided (table 22), which were designed in a way to exclude all respective reverse complement sequences from the set of K₂ sequences. As an example, if ATC is chosen as one possible k₂ element, GAT is automatically excluded from the set of k₂ elements.

For final incorporation into the primer design, the K-boxes are designed as one unit being selected to form a minimum of cross-hybridization with the 3'ends of the primes employed.

**Table 19: Optimized k₁ and k₁' sequences of 4 bp length. For example the segment side A can be employed in the right primers and B in the left primers. Furthermore, the segment side B can be employed in the right primers and A in the left primers**

| **Primer side** | **Numer** | **k₁ - or k₁' sequence** | **Melting temperature** |
|---|---|---|---|
| A | 1 | CTGA | 12 |
| A | 2 | AGTG | 12 |
| A | 3 | CAAC | 12 |
| A | 4 | GGAA | 12 |
| A | 5 | GTCA | 12 |
| A | 6 | AAGC | 12 |
| A | 7 | ATTA | 8 |
| A | 8 | AGCC | 14 |
| A | 9 | CGAG | 14 |
| A | 10 | AGGA | 12 |
| A | 11 | TAGA | 10 |
| B | 1 | GCGA | 14 |
| B | 2 | ACGG | 14 |
| B | 3 | CGTA | 12 |
| B | 4 | ACTC | 12 |
| B | 5 | CTTC | 12 |
| B | 6 | ACCA | 12 |
| B | 7 | GCAC | 14 |
| B | 8 | GACC | 14 |
| B | 9 | ATAC | 10 |
| B | 10 | CGGC | 16 |
| B | 11 | GATA | 10 |

**Table 20: Optimized k₁ and k₁' sequences of 5 bp length. For example the segment side A can be employed in the right primers and B in the left primers. Furthermore, the segment side B can be employed in the right primers and A in the left primers**

| **Primer side** | **Number** | **k₁ - or k₁' sequence** | **Melting temperature** |
|---|---|---|---|
| A | 1 | CTCTA | 14 |
| A | 2 | ATCAG | 14 |
| A | 3 | ATTGG | 14 |
| A | 4 | ATACG | 14 |
| A | 5 | ACGCA | 16 |
| A | 6 | ACCAA | 14 |
| A | 7 | AATGC | 14 |
| A | 8 | AAGGA | 14 |
| A | 9 | TCACA | 14 |
| A | 10 | ATATA | 10 |
| A | 11 | ATGTC | 14 |
| A | 12 | AGCTG | 16 |
| A | 13 | CAACC | 16 |
| B | 1 | GTTTA | 12 |
| B | 2 | GCTCC | 18 |
| B | 3 | CTTAA | 12 |
| B | 4 | GAGGC | 18 |
| B | 5 | ACACT | 14 |
| B | 6 | AATCG | 14 |
| B | 7 | CATCA | 14 |
| B | 8 | GTAGA | 14 |
| B | 9 | CTTTC | 14 |
| B | 10 | AAGCC | 16 |
| B | 11 | AAAGT | 12 |
| B | 12 | CGGAA | 16 |
| B | 13 | CTCAC | 16 |
| B | 12 | CGGAA | 16 |
| B | 13 | CTCAC | 16 |

**Table 21: Optimized k₁ and k₁' sequence of 6 bp length. For example the segment side A can be employed in the right primers and B in the left primers. Furthermore, the segment side B can be employed in the right primers and A in the left primers**

| Primer side | Number | k₁ - or k₁' sequence | Melting temperature |
|---|---|---|---|
| A | 1 | CTCTGA | 18 |
| A | 2 | GGTTAA | 16 |
| A | 3 | GCCTTA | 18 |
| A | 4 | CGGACG | 22 |
| A | 5 | GTCAAA | 16 |
| A | 6 | GATCGA | 18 |
| A | 7 | CTTGTA | 16 |
| A | 8 | AACTTG | 16 |
| A | 9 | AATCAT | 14 |
| A | 10 | ACTATG | 16 |
| A | 11 | GCAACA | 18 |
| A | 12 | CGAAGC | 20 |
| A | 13 | GAGTCC | 20 |
| A | 14 | GGCAAC | 20 |
| A | 15 | AAATGT | 14 |
| A | 16 | CTATCA | 16 |
| B | 1 | AAGCTG | 18 |
| B | 2 | GCCCAA | 20 |
| B | 3 | ATCAGA | 16 |
| B | 4 | ACTCAG | 18 |
| B | 5 | GGTATA | 16 |
| B | 6 | AAAGGG | 18 |
| B | 7 | AATGCT | 16 |
| B | 8 | CCAAGG | 20 |
| B | 9 | ACGCGG | 22 |
| B | 10 | GACGGA | 20 |
| B | 11 | GCGCAC | 22 |
| B | 12 | GTAGAA | 16 |
| B | 13 | ACCGCA | 20 |
| B | 14 | AAACCC | 18 |
| B | 15 | AGAACT | 16 |
| B | 16 | GAGCTA | 18 |

**Table 22: Examples for k₂ and k₂' sequence of 3 bp length. For example the segment side A can be employed in the right primers and B in the left primers. Furthermore, the segment side B can be employed in the right primers and A in the left primers**

| Primer side | k₂ - or k₂' sequence |
|---|---|
| A | ACG |
| A | CCA |
| A | TTA |
| A | TCG |
| A | GGT |
| B | GAC |
| B | CGG |
| B | GTG |
| B | TGT |
| B | AAG |

## Claims

1. A method for sequencing a plurality of samples containing target nucleic acid sequences tₙ₋t_{C}-t_{V}-t_{C}'-tₙ', comprising the steps of:
a. providing a set of primers for each sample of said plurality of samples, each set of primers comprising
i. a pair of initial PCR primers comprising a left initial PCR primer having a sequence k-p_{C} and a right initial primer having a sequence k'-p_{C}', and
ii. a pair of adaptor PCR primers comprising a left adaptor PCR primer consisting of a sequence a_{L}-a_{P}-a_{K} and a right adaptor PCR primer consisting of a sequence a_{L}'-3ₚ'-3_{K}',
wherein p_{C} is the same sequence as sequence element t_{C}, p_{C}' is the reverse complimentary sequence to t_{C}', p_{C} and p_{C}' each independently from one another are 8 to 40 nucleotides in length,
k comprises a 3'-terminal sequence k₁-k₂-s, and k' comprises a 3'-terminal sequence k₁'-k₂'-s',
- k₁ and k₁' each independently from one another are a sequence 1 to 8 nucleotides in length,
- k₂ and k₂' each independently from one another are 1, 2, 3, 4 or 5 nucleotides in length,
- S and S' are mismatch sequences selected not to form a continuous hybrid sequence with sequence element tₙ and tₙ' , and s and s' are independently 1, 2, 3 or 4 nucleotides in length,
a_{K} is the same sequence as sequence element k₁, a_{P}-a_{K} hybridize to a contiguous sequence on sequence element k, a_{K}' is the same sequence as sequence element k₁', a_{P}'-a_{K}' hybridize to a contiguous sequence on sequence element k', k and k' each independently from one another are a sequence 10 to 40 nucleotides in length, and wherein for all sets of primers, all sequence elements p_{C} are the same; all sequence elements p_{C}' are the same; all sequence elements a_{L}-a_{P} are the same; and all sequence elements a_{L}'- a_{P}' are the same, and each set of primers is **characterized by** a different combination of k₁ and k₁' from any other set of primers; and each set of primers is **characterized by** a different combination of k₂ and k₂' from any other set of primers,
b. and further comprising the step of obtaining a set of readout sequences from each of said samples by the steps of:
i. effecting a first amplification step, whereby said target nucleic acid sequence is amplified using said set of initial PCR primers, yielding a first amplificate;
ii. effecting a second amplification step, whereby said first amplificate is amplified using said set of adaptor PCR primers, yielding a second amplificate;
iii. sequencing said second amplificate, yielding a set of readout sequences;
iv. aligning said set of readout sequences to sequence elements k and k' comprised in said set of initial primers, and
v. assigning a measure of 0 or 1 of contamination to said set of readout sequences, wherein complete alignment of each member of said set of readout sequences to said sequence element k or k' is assigned a measure 0 of contamination (= no contamination), and incomplete alignment of said set of readout sequences to said sequence element k or k' is assigned a measure 1 of contamination (= is contaminated).

2. The method for sequencing a target nucleic acid sequence according to claim 1, wherein said left initial primer, said right initial primer, said left adaptor primer and/or said right adaptor primer are **characterized by** a nuclease resistant nucleotide or a nuclease resistant internucleosidic bond on the 3' terminus of said primer.

3. A collection of sets of primers for use in a method for sequencing a plurality of samples containing target nucleic sequences tₙ-t_{C}-t_{V}-t_{C}'-tₙ', each set of primers of said collection comprising
i. a pair of initial PCR primers comprising a left initial PCR primer having a sequence k-p_{C} and a right initial primer having a sequence k'-p_{C}', and
ii. a pair of adaptor PCR primers comprising a left adaptor PCR primer consisting of a sequence a_{L}-a_{P}-a_{K} and a right adaptor PCR primer consisting of a sequence a_{L}'- a_{P}'-a_{K}',
wherein p_{C} is the same sequence as sequence element t_{C}, p_{C}' is the reverse complimentary sequence to t_{C}', p_{C} and p_{C}' each independently from one another are 8 to 40 nucleotides in length,
k comprises a 3'-terminal sequence k₁-k₂-s, and k' comprises a 3'-terminal sequence k₁'-k₂'-s',
- k₁ and k₁' each independently from one another are a sequence 1 to 8 nucleotides in length,
- k₂ and k₂' each independently from one another are 1, 2, 3, 4 or 5 nucleotides in length,
- S and S' are mismatch sequences selected not to form a continuous hybrid sequence with sequence element tₙ and tₙ', and s and s' are independently 1, 2, 3 or 4 nucleotides in length,
a_{P}-a_{K} hybridize to a contiguous sequence on sequence element k, aₖ is the same sequence as sequence element k₁, a_{K}' is the same sequence as sequence element k₁', a_{P}'-a_{K}' hybridize to a contiguous sequence on sequence element k', k and k' each independently from one another are a sequence 10 to 40 nucleotides in length, a_{L} and a_{L}' independently from one another can be any sequence, and wherein for all sets of primers comprised within said collection, all sequence elements p_{C} are the same; all sequence elements p_{C}' are the same; all sequence elements a_{P} are the same; all sequence elements a_{P}' are the same; and each set of primers is **characterized by** a different combination of k₁ and k₁' from any other set of primers; and each set of primers is **characterized by** a different combination of k₂ and k₂' from any other set of primers.

4. A collection of sets of primers according to claim 3, wherein sequence element p_{C} is selected from table 1 and sequence element p_{C}' is selected from table 2.

5. A primer library comprising a plurality of collections of sets of primers according to claim 3, wherein each collection is **characterized by** a different combination of p_{C} and p_{C}'.

## Patentansprüche

1. Ein Verfahren zur Sequenzierung einer Vielzahl von Ziel-Nukleinsäuresequenzen tn-tₙ-t_{C}-t_{V}-t_{C}'-tₙ' enthaltenden Proben, welches die Schritte enthält:
a. ein Primer-Set wird für jede Probe der Vielzahl von Proben zur Verfügung gestellt, wobei jedes Primer-Set
i. ein Paar initialer PCR-Primer, welches einen linken initialen PCR-Primer umfasst, der die Sequenz k-p_{C} hat, und einen rechten initialen Primer umfasst, der die Sequenz k'-p_{C}' hat, und
ii. ein Paar Adapter-PCR-Primer, welches einen linken Adapter-PCR-Primer umfasst, der aus der Sequenz a_{L}-a_{P}-a_{K} besteht, und einen rechten Adapter-PCR-Primer umfasst, der aus der Sequenz a_{L}'-a_{P}'-a_{K}', besteht
umfasst,
k eine 3' terminale Sequenz k₁-k₂-s umfasst und k' eine 3' terminale Sequenz k₁'-k₂'-S' umfasst,
- k₁ and k₁' jeweils unabhängig voneinander eine Sequenz von 1 bis 8 Nukleotide Länge sind,
- k₂ and k₂' jeweils unabhängig voneinander 1, 2, 3, 4 oder 5 Nukleotide lang sind,
- S und S' nicht paarende Sequenzen sind, welche so ausgewählt sind, dass sie keine durchgehende Hybridsequenz mit dem Sequenzelement tₙ und tₙ' formen, und s und s' unabhängig voneinander miteinander 1, 2, 3 oder 4 Nukleotide lang sind,
a_{K} das selbe Sequenzelement ist wie das Sequenzelement k₁, a_{P}-a_{K} auf einer durchgehenden Sequenz auf Sequenzelement k hybridisieren, a_{K}' das selbe Sequenzelement ist wie das Sequenzelement k₁', a_{P}-a_{K}' mit einer durchgehenden Sequenz auf Sequenzelement k' hybridisieren, k und k' jeweils unabhängig voneinander eine Sequenz von 10 bis 40 Nukleotiden Länge sind und wobei für alle Primer-Sets alle Sequenzelemente p_{C} die selben sind, alle Sequenzelemente p_{C}' dieselben sind, alle Sequenzeiemente a_{L}-3_{P} die selben sind und alle Sequenzelemente a_{L}'- a_{P}' dieselben sind, und jedes Primer-Set durch eine andere Kombination von k₁ und k₁' von allen anderen Primer- Sets unterschieden ist, und jedes Primer-Set von allen anderen Primer-Sets durch eine andere Kombination von k₂ und k₂' unterschieden ist,
b. und weiterhin aufweisend den Schritt, dass ein Set von ausgelesenen Sequenzen von jeder der genannten Proben durch die folgenden Schritte gewonnen wird:
I. Ausführen eines ersten Amplifikationsschrittes, wodurch die Ziel-Nukleinsäuresequenz unter Gebrauch genannter initialer PCR- Primer amplifiziert wird, wodurch ein erstes Amplifikat gewonnen wird;
II. Ausführen eines zweiten Amplifikationsschrittes, wobei besagtes erstes Amplifikat unter Verwendung des besagten Sets von Adapter PCR-Primern amplifiziert wird, wodurch ein zweites Amplifikat gewonnen wird;
III. Sequenzierung des besagten zweiten Amplifikats, wodurch ein Set von Lesesequenzen gewonnen wird;
IV. Sequenzvergleich besagter Sets von Lesesequenzen mit Sequenzelementen k und k' welche in besagtem Set von initialen Primern enthalten sind, und
V. Zuordnung eines Maßes von 0 oder 1 an Kontamination zu besagtem Set von Lesesequenzen, wobei vollkommene Sequenzgleichheit jedes Mitglieds besagten Sets von Lesesequenzen mit Sequenzelement k oder k' ein Maß 0 der Kontamination zugeordnet ist (= keine Kontamination), und nicht vollständige Sequenzgleichheit besagten Sets von Lesesequenzen mit besagtem Sequenzelement k oder k' ein Maß 1 an Kontamination zugeordnet ist (= ist kontaminiert).

2. Das Verfahren zur Sequenzierung einer Ziel-Nukleinsäuresequenz gemäß Anspruch 1, wobei besagter linker initialer Primer, besagter rechter initialer Primer, besagter linker Adapter Primer und/oder besagter rechter Adapter Primer durch ein Nukleaseresistentes Nukleotid oder eine Nuklease-resistente interne nukleosidische Bindung am 3'-Terminus des besagten Primers gekennzeichnet sind.

3. Eine Sammlung von Primer-Sets zum Gebrauch in einem Verfahren zur Sequenzierung einer Vielzahl von Ziel-Nukleinsäuresequenzen tₙ-tₙ-t_{C}-t_{V}-t_{C}'-tₙ' enthaltenden Proben, wobei jedes Primer-Set der Sammlung
i. ein Paar initialer PCR-Primer, welches einen linken initialen PCR-Primer umfasst, der die Sequenz k-p_{C} hat, und einen rechten initialen Primer umfasst, der die Sequenz k'-p_{C}' hat, und
ii. ein Paar Adapter-PCR-Primer, welches einen linken Adapter-PCR-Primer umfasst, der aus der Sequenz a_{L}-a_{P}-a_{K} besteht, und einen rechten Adapter-PCR-Primer umfasst, der aus der Sequenz a_{L}'-a_{P}'-a_{K}', besteht umfasst,
wobei p_{C} das selbe Sequenzelement ist wie t_{C}, p_{C}' die revers komplementäre Sequenz zu t_{C}' ist, p_{C} und p_{C}' jeweils unabhängig voneinander eine Sequenz von 8 bis 40 Nukleotiden Länge sind,
k eine 3'-terminale Sequenz k₁-k₂-s umfasst, und k' eine 3'-terminale Sequenz k₁'-k₂'-s' umfasst,
- k₁ and k₁' jeweils unabhängig voneinander eine Sequenz von 1 bis 8 Nukleotide Länge sind,
- k₂ and k₂' jeweils unabhängig voneinander 1, 2, 3, 4 oder 5 Nukleotide lang sind,
- S und s' nicht paarende Sequenzen sind, welche so ausgewählt sind, dass sie keine durchgehende Hybridsequenz mit dem Sequenzelement tₙ und tₙ' formen, und s und s' unabhängig voneinander miteinander 1, 2, 3 oder 4 Nukleotide lang sind,
a_{P}-a_{K} auf einer durchgehenden Sequenz auf Sequenzelement k hybridisieren, a_{K} das selbe Sequenzelement ist wie das Sequenzelement k₁, a_{K}' das selbe Sequenzelement ist wie das Sequenzelement k₁', a_{P}-a_{K}' mit einer durchgehenden Sequenz auf Sequenzelement k' hybridisieren, kund k' jeweils unabhängig voneinander eine Sequenz von 10 bis 40 Nukleotiden Länge sind, a_{L} und a_{L}' unabhängig voneinander jede Sequenz sein können, und wobei für alle in dieser Sammlung umfassten Sets an Primern alle Sequenzelemente p_{C} die selben sind, alle Sequenzelemente p_{C}' die selben sind; alle Sequenzelemente a_{P} die selben sind; alle Sequenzelemente a_{P}' die selben sind; und jedes Primer-Set durch eine unterschiedliche Kombination von k₁ und k₁' von jedem anderen Primer-Set unterschieden ist; und jedes Primer-Set durch eine unterschiedliche Kombination von k₂ und k₂' von jedem anderen Primer-Set unterschieden ist.

4. Eine Sammlung von Primer-Sets gemäß Anspruch 3, wobei Sequenzelement p_{C} aus Tafel 1 ausgewählt ist und Sequenzelement p_{C}' aus Tafel 2 ausgewählt ist.

5. Eine Primerbibliothek umfassend eine Vielzahl von Sammlungen von Primer-Sets gemäß Anspruch 3, wobei jede Sammlung durch eine andere Kombination von p_{C} and p_{C}' gekennzeichnet ist.

## Revendications

1. Procédé de séquençage d'une pluralité d'échantillons contenant des séquences d'acide nucléique cibles tₙ-t_{C}-t_{V}-t_{C}'-tₙ', comprenant les étapes suivantes :
a. mise à disposition d'un ensemble d'amorces pour chaque échantillon de ladite pluralité d'échantillons, chaque ensemble d'amorces comprenant
i. une paire d'amorces PCR initiales comprenant une amorce PCR initiale gauche présentant une séquence k-p_{C} et une amorce initiale droite présentant une séquence k'-p_{C}', et
ii. une paire d'amorces PCR adaptateurs comprenant une amorce PCR adaptateur gauche se composant d'un séquence a_{L}-a_{P}-a_{K} et une amorce PCR adaptateur droite se composant d'une séquence a_{L}'-a_{P}'-a_{K}',
dans lequel p_{C} est la même séquence que l'élément de séquence t_{C}, p_{C}' est la séquence complémentaire inverse de t_{C}', p_{C} et p_{C}' ont chacune, indépendamment l'une de l'autre, une longueur de 8 à 40 nucléotides,
k comprend une séquence terminale 3' k₁-k₂-s, et k' comprend une séquence terminale 3' k₁'-k₂'-s',
- k₁ et k₁' sont chacune, indépendamment l'une de l'autre, une séquence de 1 à 8 nucléotides de longueur,
- k₂ et k₂' ont chacune, indépendamment l'une de l'autre, une longueur de 1, 2, 3, 4 ou 5 nucléotides,
- S et s' sont des séquences mésappariées sélectionnées de manière à ne pas former une séquence hybride continue avec l'élément de séquence tₙ et tₙ', et s et s' ont indépendamment l'une de l'autre une longueur de 1, 2, 3 ou 4 nucléotides,
a_{K} est la même séquence que l'élément de séquence k₁, a_{P}-a_{K} s'hybrident à une séquence contiguë sur l'élément de séquence k, a_{K}' est la même séquence que l'élément de séquence k₁', a_{P}'-a_{K}' s'hybrident à une séquence contiguë sur l'élément de séquence k', k et k' sont chacune, indépendamment l'une de l'autre, une séquence d'une longueur de 10 à 40 nucléotides, et dans lequel, pour tous les ensembles d'amorces, tous les éléments de séquence p_{C} sont les même ; tous les éléments de séquence p_{C}' sont les mêmes ; tous les éléments de séquence a_{L}-a_{P} sont les mêmes ; et tous les éléments de séquence a_{L}'- a_{P}' sont les mêmes, et chaque ensemble d'amorces est **caractérisé par** une combinaison différente de k₁ et k₁' de n'importe quel autre ensemble d'amorces ; et chaque ensemble d'amorces est **caractérisé par** une combinaison différente de k₂ et k₂' de n'importe quel autre ensemble d'amorces,
b. et comprenant en outre l'étape consistant à obtenir un ensemble de séquences de lecture de chacun desdits échantillons par les étapes suivantes :
i. réalisation d'une première étape d'amplification au cours de laquelle ladite séquence d'acide nucléique cible est amplifiée au moyen dudit ensemble d'amorces PCR initiales pour donner un premier amplificat ;
ii. réalisation d'une deuxième étape d'amplification au cours de laquelle ledit premier amplificat est amplifié au moyen dudit ensemble d'amorces PCR adaptateurs pour donner un deuxième amplificat ;
iii. séquençage dudit deuxième amplificat pour donner un ensemble de séquences de lecture ;
iv. alignement dudit ensemble de séquences de lecture sur les éléments de séquence k et k' compris dans ledit ensemble d'amorces initiales, et
v. attribution d'une valeur 0 ou 1 de contamination audit ensemble de séquences de lecture, l'alignement complet de chaque maillon dudit ensemble de séquences de lecture audit élément de séquence k ou k' étant affecté à une valeur 0 de contamination (= pas de contamination), et un alignement incomplet dudit ensemble de séquences de lecture audit élément de séquence k ou k' étant affecté à une valeur 1 de contamination (= est contaminé).

2. Procédé de séquençage d'une séquence d'acide nucléique cible selon la revendication 0, dans lequel ladite amorce initiale gauche, ladite amorce initiale droite, ladite amorce adaptateur gauche et/ou ladite amorce adaptateur droite sont **caractérisées par** un nucléotide résistant aux nucléases ou une liaison internucléosidique résistante aux nucléases au terminal 3' de ladite amorce.

3. Collection d'ensembles d'amorces pour utilisation dans un procédé de séquençage d'une pluralité d'échantillons contenant des séquences d'acide nucléique cibles tₙ-t_{C}-t_{V}-t_{C}'-tₙ', chaque ensemble d'amorces de ladite collection comprenant
i. une paire d'amorces PCR initiales comprenant une amorce PCR initiale gauche présentant une séquence k-p_{C} et une amorce initiale droite présentant une séquence k'-p_{C}', et
ii. une paire d'amorces PCR adaptateurs comprenant une amorce PCR adaptateur gauche se composant d'une séquence a_{L}-a_{P}-a_{K} et une amorce PCR adaptateur droite se composant d'une séquence a_{L}'-a_{P}'-a_{K}',
dans lequel p_{C} est la même séquence que l'élément de séquence t_{C}, p_{C}' est la séquence complémentaire inverse de t_{C}', p_{C} et p_{C}' ont chacune, indépendamment l'une de l'autre, une longueur de 8 à 40 nucléotides,
k comprend une séquence terminale 3' k₁-k₂-s, et k' comprend une séquence terminale 3' k₁'-k₂'-s',
- k₁ et k₁' sont chacune, indépendamment l'une de l'autre, une séquence de 1 à 8 nucléotides de longueur,
- k₂ et k₂' ont chacune, indépendamment l'une de l'autre, une longueur de 1, 2, 3, 4 ou 5 nucléotides,
- S et s' sont des séquences mésappariées sélectionnées de manière à ne pas former une séquence hybride continue avec l'élément de séquence tₙ et tₙ', et s et s' ont indépendamment l'une de l'autre une longueur de 1, 2, 3 ou 4 nucléotides,
a_{P}-a_{K} s'hybrident à une séquence contiguë sur l'élément de séquence k, a_{K} est la même séquence que l'élément de séquence k₁, a_{K}' est la même séquence que l'élément de séquence k₁', a_{P}'-a_{K}' s'hybrident à une séquence contiguë sur l'élément de séquence k', k et k' sont chacune, indépendamment l'une de l'autre, une séquence d'une longueur de 10 à 40 nucléotides, a_{L} et a_{L}' peuvent être, indépendamment l'une de l'autre, n'importe quelle séquence, et dans lequel pour tous les ensembles d'amorces compris au sein de ladite collection, tous les éléments de séquence p_{C} sont les mêmes ; tous les éléments de séquence p_{C}' sont les mêmes ; tous les éléments de séquence a_{P} sont les mêmes ; tous les éléments de séquence a_{P}' sont les mêmes ; et chaque ensemble d'amorces est **caractérisé par** une combinaison différente de k₁ et k₁' de n'importe quel autre ensemble d'amorces ; et chaque ensemble d'amorces est **caractérisé par** une combinaison différente de k₂ et k₂' de n'importe quel autre ensemble d'amorces,

4. Collection d'ensembles d'amorces selon la revendication 3, dans laquelle l'élément de séquence p_{C} est sélectionné dans la table 1 et élément de séquence p_{C}' est sélectionné dans la table 2.

5. Bibliothèque d'amorces comprenant une pluralité de collections d'ensembles d'amorces selon la revendication 3, dans laquelle chaque collection est **caractérisée par** une combinaison différente de p_{C} et p_{C}'.


### SEQUENCE LISTING

<110> HS Diagnomics GmbH
<120> Methods and primer sets for high throughput PCR sequencing
<130> 12199315.8
<150> EP12199315.8
   <151> 2012-12-23
<160> 192
<170> BiSSAP 1.2
<210> 1
   <211> 28
   <212> DNA
   <213> Artificial Sequence
<220>
   <221> source
   <222> 1..28
   <223> /organism="Artificial Sequence"
   /note="Primer for PCR"
   /mol_type="unassigned DNA"
<400> 1
   aatttcactc tgaagatccg gtccacaa 28
<210> 2
   <211> 28
   <212> DNA
   <213> Artificial Sequence
<220>
   <221> source
   <222> 1..28
   <223> /organism="Artificial Sequence"
   /note="Primer for PCR"
   /mol_type="unassigned DNA"
<400> 2
   cacttaaatc ttcacatcaa ttccctgg 28
<210> 3
   <211> 25
   <212> DNA
   <213> Artificial Sequence
<220>
   <221> source
   <222> 1..25
   <223> /organism="Artificial Sequence"
   /note="Primer for PCR"
   /mol_type="unassigned DNA"
<400> 3
   ttaaaccttc acctacacgc cctgc 25
<210> 4
   <211> 25
   <212> DNA
   <213> Artificial Sequence
<220>
   <221> source
   <222> 1..25
   <223> /organism="Artificial Sequence"
   /note="Primer for PCR"
   /mol_type="unassigned DNA"
<400> 4
   ttattccttc acctacacac cctgc 25
<210> 5
   <211> 27
   <212> DNA
   <213> Artificial Sequence
<220>
   <221> source
   <222> 1..27
   <223> /organism="Artificial Sequence"
   /note="Primer for PCR"
   /mol_type="unassigned DNA"
<400> 5
   aactctgaga tgaatgtgag caccttg 27
<210> 6
   <211> 27
   <212> DNA
   <213> Artificial Sequence
<220>
   <221> source
   <222> 1..27
   <223> /organism="Artificial Sequence"
   /note="Primer for PCR"
   /mol_type="unassigned DNA"
<400> 6
   tgctctgaga tgaatgtgag tgccttg 27
<210> 7
   <211> 27
   <212> DNA
   <213> Artificial Sequence
<220>
   <221> source
   <222> 1..27
   <223> /organism="Artificial Sequence"
   /note="Primer for PCR"
   /mol_type="unassigned DNA"
<400> 7
   agctctgagc tgaatgtgaa cgccttg 27
<210> 8
   <211> 22
   <212> DNA
   <213> Artificial Sequence
<220>
   <221> source
   <222> 1..22
   <223> /organism="Artificial Sequence"
   /note="Primer for PCR"
   /mol_type="unassigned DNA"
<400> 8
   tcgctcaggc tggagtcggc tg 22
<210> 9
   <211> 25
   <212> DNA
   <213> Artificial Sequence
<220>
   <221> source
   <222> 1..25
   <223> /organism="Artificial Sequence"
   /note="Primer for PCR"
   /mol_type="unassigned DNA"
<400> 9
   attttcctgg ggttggagtc ggctg 25
<210> 10
   <211> 25
   <212> DNA
   <213> Artificial Sequence
<220>
   <221> source
   <222> 1..25
   <223> /organism="Artificial Sequence"
   /note="Primer for PCR"
   /mol_type="unassigned DNA"
<400> 10
   ttccccctca cgttggcgtc tgctg 25
<210> 11
   <211> 22
   <212> DNA
   <213> Artificial Sequence
<220>
   <221> source
   <222> 1..22
   <223> /organism="Artificial Sequence"
   /note="Primer for PCR"
   /mol_type="unassigned DNA"
<400> 11
   ccgctcaggc tgctgtcggc tg 22
<210> 12
   <211> 25
   <212> DNA
   <213> Artificial Sequence
<220>
   <221> source
   <222> 1..25
   <223> /organism="Artificial Sequence"
   /note="Primer for PCR"
   /mol_type="unassigned DNA"
<400> 12
   ttcccgctca ggctggagtt ggctg 25
<210> 13
   <211> 25
   <212> DNA
   <213> Artificial Sequence
<220>
   <221> source
   <222> 1..25
   <223> /organism="Artificial Sequence"
   /note="Primer for PCR"
   /mol_type="unassigned DNA"
<400> 13
   ttccccctca agctggagtc agctg 25
<210> 14
   <211> 25
   <212> DNA
   <213> Artificial Sequence
<220>
   <221> source
   <222> 1..25
   <223> /organism="Artificial Sequence"
   /note="Primer for PCR"
   /mol_type="unassigned DNA"
<400> 14
   ttcccactca ggctggtgtc ggctg 25
<210> 15
   <211> 25
   <212> DNA
   <213> Artificial Sequence
<220>
   <221> source
   <222> 1..25
   <223> /organism="Artificial Sequence"
   /note="Primer for PCR"
   /mol_type="unassigned DNA"
<400> 15
   ttcccgctca ggctggagtc agctg 25
<210> 16
   <211> 25
   <212> DNA
   <213> Artificial Sequence
<220>
   <221> source
   <222> 1..25
   <223> /organism="Artificial Sequence"
   /note="Primer for PCR"
   /mol_type="unassigned DNA"
<400> 16
   tccactctga agttccagcg cacac 25
<210> 17
   <211> 25
   <212> DNA
   <213> Artificial Sequence
<220>
   <221> source
   <222> 1..25
   <223> /organism="Artificial Sequence"
   /note="Primer for PCR"
   /mol_type="unassigned DNA"
<400> 17
   tccactctga cgatccagcg cacac 25
<210> 18
   <211> 25
   <212> DNA
   <213> Artificial Sequence
<220>
   <221> source
   <222> 1..25
   <223> /organism="Artificial Sequence"
   /note="Primer for PCR"
   /mol_type="unassigned DNA"
<400> 18
   tctactctga agatccagcg cacag 25
<210> 19
   <211> 25
   <212> DNA
   <213> Artificial Sequence
<220>
   <221> source
   <222> 1..25
   <223> /organism="Artificial Sequence"
   /note="Primer for PCR"
   /mol_type="unassigned DNA"
<400> 19
   tccactctga agatccagcg cacag 25
<210> 20
   <211> 25
   <212> DNA
   <213> Artificial Sequence
<220>
   <221> source
   <222> 1..25
   <223> /organism="Artificial Sequence"
   /note="Primer for PCR"
   /mol_type="unassigned DNA"
<400> 20
   tccactctga cgatccagcg cacag 25
<210> 21
   <211> 25
   <212> DNA
   <213> Artificial Sequence
<220>
   <221> source
   <222> 1..25
   <223> /organism="Artificial Sequence"
   /note="Primer for PCR"
   /mol_type="unassigned DNA"
<400> 21
   tccactctga cgattcagcg cacag 25
<210> 22
   <211> 25
   <212> DNA
   <213> Artificial Sequence
<220>
   <221> source
   <222> 1..25
   <223> /organism="Artificial Sequence"
   /note="Primer for PCR"
   /mol_type="unassigned DNA"
<400> 22
   tccactctga agatccagcg cacac 25
<210> 23
   <211> 25
   <212> DNA
   <213> Artificial Sequence
<220>
   <221> source
   <222> 1..25
   <223> /organism="Artificial Sequence"
   /note="Primer for PCR"
   /mol_type="unassigned DNA"
<400> 23
   tccaccttgg agatccagcg cacag 25
<210> 24
   <211> 27
   <212> DNA
   <213> Artificial Sequence
<220>
   <221> source
   <222> 1..27
   <223> /organism="Artificial Sequence"
   /note="Primer for PCR"
   /mol_type="unassigned DNA"
<400> 24
   cactctgaac taaacctgag ctctctg 27
<210> 25
   <211> 25
   <212> DNA
   <213> Artificial Sequence
<220>
   <221> source
   <222> 1..25
   <223> /organism="Artificial Sequence"
   /note="Primer for PCR"
   /mol_type="unassigned DNA"
<400> 25
   ctccccctca ctctggagtc tgctg 25
<210> 26
   <211> 25
   <212> DNA
   <213> Artificial Sequence
<220>
   <221> source
   <222> 1..25
   <223> /organism="Artificial Sequence"
   /note="Primer for PCR"
   /mol_type="unassigned DNA"
<400> 26
   ttccccctca ctctggagtc agcta 25
<210> 27
   <211> 25
   <212> DNA
   <213> Artificial Sequence
<220>
   <221> source
   <222> 1..25
   <223> /organism="Artificial Sequence"
   /note="Primer for PCR"
   /mol_type="unassigned DNA"
<400> 27
   ttcctcctca ctctggagtc cgcta 25
<210> 28
   <211> 25
   <212> DNA
   <213> Artificial Sequence
<220>
   <221> source
   <222> 1..25
   <223> /organism="Artificial Sequence"
   /note="Primer for PCR"
   /mol_type="unassigned DNA"
<400> 28
   tccactctca agatccagcc tgcag 25
<210> 29
   <211> 25
   <212> DNA
   <213> Artificial Sequence
<220>
   <221> source
   <222> 1..25
   <223> /organism="Artificial Sequence"
   /note="Primer for PCR"
   /mol_type="unassigned DNA"
<400> 29
   tccactctca agatccagcc tgcaa 25
<210> 30
   <211> 25
   <212> DNA
   <213> Artificial Sequence
<220>
   <221> source
   <222> 1..25
   <223> /organism="Artificial Sequence"
   /note="Primer for PCR"
   /mol_type="unassigned DNA"
<400> 30
   tccactctga agatccagcc ctcag 25
<210> 31
   <211> 28
   <212> DNA
   <213> Artificial Sequence
<220>
   <221> source
   <222> 1..28
   <223> /organism="Artificial Sequence"
   /note="Primer for PCR"
   /mol_type="unassigned DNA"
<400> 31
   cattctgaac tgaacatgag ctccttgg 28
<210> 32
   <211> 25
   <212> DNA
   <213> Artificial Sequence
<220>
   <221> source
   <222> 1..25
   <223> /organism="Artificial Sequence"
   /note="Primer for PCR"
   /mol_type="unassigned DNA"
<400> 32
   tctactctga aggtgcagcc tgcag 25
<210> 33
   <211> 25
   <212> DNA
   <213> Artificial Sequence
<220>
   <221> source
   <222> 1..25
   <223> /organism="Artificial Sequence"
   /note="Primer for PCR"
   /mol_type="unassigned DNA"
<400> 33
   aacttccaat ccaggaggcc gaaca 25
<210> 34
   <211> 25
   <212> DNA
   <213> Artificial Sequence
<220>
   <221> source
   <222> 1..25
   <223> /organism="Artificial Sequence"
   /note="Primer for PCR"
   /mol_type="unassigned DNA"
<400> 34
   tgtagccttg agatccaggc tacga 25
<210> 35
   <211> 25
   <212> DNA
   <213> Artificial Sequence
<220>
   <221> source
   <222> 1..25
   <223> /organism="Artificial Sequence"
   /note="Primer for PCR"
   /mol_type="unassigned DNA"
<400> 35
   tcttccacgc tgaagatcca tcccg 25
<210> 36
   <211> 25
   <212> DNA
   <213> Artificial Sequence
<220>
   <221> source
   <222> 1..25
   <223> /organism="Artificial Sequence"

   /note="Primer for PCR"
   /mol_type="unassigned DNA"
<400> 36
   agcatcctga ggatccagca ggtag 25
<210> 37
   <211> 25
   <212> DNA
   <213> Artificial Sequence
<220>
   <221> source
   <222> 1..25
   <223> /organism="Artificial Sequence"
   /note="Primer for PCR"
   /mol_type="unassigned DNA"
<400> 37
   tttcctctca ctgtgacatc ggccc 25
<210> 38
   <211> 25
   <212> DNA
   <213> Artificial Sequence
<220>
   <221> source
   <222> 1..25
   <223> /organism="Artificial Sequence"
   /note="Primer for PCR"
   /mol_type="unassigned DNA"
<400> 38
   ttgtccactc tgacagtgac cagtg 25
<210> 39
   <211> 25
   <212> DNA
   <213> Artificial Sequence
<220>
   <221> source
   <222> 1..25
   <223> /organism="Artificial Sequence"
   /note="Primer for PCR"
   /mol_type="unassigned DNA"
<400> 39
   tgcagcctgg caatcctgtc ctcag 25
<210> 40
   <211> 26
   <212> DNA
   <213> Artificial Sequence
<220>
   <221> source
   <222> 1..26
   <223> /organism="Artificial Sequence"
   /note="Primer for PCR"
   /mol_type="unassigned DNA"
<400> 40
   ttctccctgt ccctagagtc tgccat 26
<210> 41
   <211> 25
   <212> DNA
   <213> Artificial Sequence
<220>
   <221> source
   <222> 1..25
   <223> /organism="Artificial Sequence"
   /note="Primer for PCR"
   /mol_type="unassigned DNA"
<400> 41
   tttcccctga ccctggagtc tgcca 25
<210> 42
   <211> 25
   <212> DNA
   <213> Artificial Sequence
<220>
   <221> source
   <222> 1..25
   <223> /organism="Artificial Sequence"
   /note="Primer for PCR"
   /mol_type="unassigned DNA"
<400> 42
   ttccccctga tcctggagtc gccca 25
<210> 43
   <211> 25
   <212> DNA
   <213> Artificial Sequence
<220>
   <221> source
   <222> 1..25
   <223> /organism="Artificial Sequence"
   /note="Primer for PCR"
   /mol_type="unassigned DNA"
<400> 43
   ttctccctga ttctggagtc cgcca 25
<210> 44
   <211> 28
   <212> DNA
   <213> Artificial Sequence
<220>
   <221> source
   <222> 1..28
   <223> /organism="Artificial Sequence"
   /note="Primer for PCR"
   /mol_type="unassigned DNA"
<400> 44
   acattctcaa ctctgactgt gagcaaca 28
<210> 45
   <211> 28
   <212> DNA
   <213> Artificial Sequence
<220>
   <221> source
   <222> 1..28
   <223> /organism="Artificial Sequence"
   /note="Primer for PCR"
   /mol_type="unassigned DNA"
<400> 45
   cggcagttca tcctgagttc taagaagc 28
<210> 46
   <211> 26
   <212> DNA
   <213> Artificial Sequence
<220>
   <221> source
   <222> 1..26
   <223> /organism="Artificial Sequence"
   /note="Primer for PCR"
   /mol_type="unassigned DNA"
<400> 46
   ttacctacaa ctgtgagtct ggtgcc 26
<210> 47
   <211> 25
   <212> DNA
   <213> Artificial Sequence
<220>
   <221> source
   <222> 1..25
   <223> /organism="Artificial Sequence"
   /note="Primer for PCR"
   /mol_type="unassigned DNA"
<400> 47
   cctacaacgg ttaacctggt ccccg 25
<210> 48
   <211> 25
   <212> DNA
   <213> Artificial Sequence
<220>
   <221> source
   <222> 1..25
   <223> /organism="Artificial Sequence"
   /note="Primer for PCR"
   /mol_type="unassigned DNA"
<400> 48
   cctacaacag tgagccaact tccct 25
<210> 49
   <211> 26
   <212> DNA
   <213> Artificial Sequence
<220>
   <221> source
   <222> 1..26
   <223> /organism="Artificial Sequence"
   /note="Primer for PCR"
   /mol_type="unassigned DNA"
<400> 49
   cccaagacag agagctgggt tccact 26
<210> 50
   <211> 25
   <212> DNA
   <213> Artificial Sequence
<220>
   <221> source
   <222> 1..25
   <223> /organism="Artificial Sequence"
   /note="Primer for PCR"
   /mol_type="unassigned DNA"
<400> 50
   ttacctagga tggagagtcg agtcc 25
<210> 51
   <211> 25
   <212> DNA
   <213> Artificial Sequence
<220>
   <221> source
   <222> 1..25
   <223> /organism="Artificial Sequence"
   /note="Primer for PCR"
   /mol_type="unassigned DNA"
<400> 51
   cctgtcacag tgagcctggt cccgt 25
<210> 52
   <211> 23
   <212> DNA
   <213> Artificial Sequence
<220>
   <221> source
   <222> 1..23
   <223> /organism="Artificial Sequence"
   /note="Primer for PCR"
   /mol_type="unassigned DNA"
<400> 52
   cctagcacgg tgagccgtgt ccc 23
<210> 53
   <211> 25
   <212> DNA
   <213> Artificial Sequence
<220>
   <221> source
   <222> 1..25
   <223> /organism="Artificial Sequence"
   /note="Primer for PCR"
   /mol_type="unassigned DNA"
<400> 53
   ttacccagta cggtcagcct agagc 25
<210> 54
   <211> 23
   <212> DNA
   <213> Artificial Sequence
<220>
   <221> source
   <222> 1..23
   <223> /organism="Artificial Sequence"
   /note="Primer for PCR"
   /mol_type="unassigned DNA"
<400> 54
   agcactgtca gccgggtgcc tgg 23
<210> 55
   <211> 23
   <212> DNA
   <213> Artificial Sequence
<220>
   <221> source
   <222> 1..23
   <223> /organism="Artificial Sequence"
   /note="Primer for PCR"
   /mol_type="unassigned DNA"
<400> 55
   agcactcaga gccgggtccc ggc 23
<210> 56
   <211> 22
   <212> DNA
   <213> Artificial Sequence
<220>
   <221> source
   <222> 1..22
   <223> /organism="Artificial Sequence"
   /note="Primer for PCR"
   /mol_type="unassigned DNA"
<400> 56
   ccgagcacca ggagccgcgt gc 22
<210> 57
   <211> 23
   <212> DNA
   <213> Artificial Sequence
<220>
   <221> source
   <222> 1..23
   <223> /organism="Artificial Sequence"
   /note="Primer for PCR"

   /mol_type="unassigned DNA"
<400> 57
   agcacggtca gcctgctgcc ggc 23
<210> 58
   <211> 23
   <212> DNA
   <213> Artificial Sequence
<220>
   <221> source
   <222> 1..23
   <223> /organism="Artificial Sequence"
   /note="Primer for PCR"
   /mol_type="unassigned DNA"
<400> 58
   gtgaccgtga gcctggtgcc cgg 23
<210> 59
   <211> 37
   <212> DNA
   <213> Artificial Sequence
<220>
   <221> source
   <222> 1..37
   <223> /organism="Artificial Sequence"
   /note="Primer for PCR"
   /mol_type="unassigned DNA"
<400> 59
   atctacactc tttccctaca cgacgctctt ccgatct 37
<210> 60
   <211> 36
   <212> DNA
   <213> Artificial Sequence
<220>
   <221> source
   <222> 1..36
   <223> /organism="Artificial Sequence"
   /note="Primer for PCR"
   /mol_type="unassigned DNA"
<400> 60
   tctacactct ttccctacac gacgctcttc cgatct 36
<210> 61
   <211> 35
   <212> DNA
   <213> Artificial Sequence
<220>
   <221> source
   <222> 1..35
   <223> /organism="Artificial Sequence"
   /note="Primer for PCR"
   /mol_type="unassigned DNA"
<400> 61
   ctacactctt tccctacacg acgctcttcc gatct 35
<210> 62
   <211> 34
   <212> DNA
   <213> Artificial Sequence
<220>
   <221> source
   <222> 1..34
   <223> /organism="Artificial Sequence"
   /note="Primer for PCR"
   /mol_type="unassigned DNA"
<400> 62
   tacactcttt ccctacacga cgctcttccg atct 34
<210> 63
   <211> 33
   <212> DNA
   <213> Artificial Sequence
<220>
   <221> source
   <222> 1..33
   <223> /organism="Artificial Sequence"
   /note="Primer for PCR"
   /mol_type="unassigned DNA"
<400> 63
   acactctttc cctacacgac gctcttccga tct 33
<210> 64
   <211> 32
   <212> DNA
   <213> Artificial Sequence
<220>
   <221> source
   <222> 1..32
   <223> /organism="Artificial Sequence"
   /note="Primer for PCR"
   /mol_type="unassigned DNA"
<400> 64
   cactctttcc ctacacgacg ctcttccgat ct 32
<210> 65
   <211> 31
   <212> DNA
   <213> Artificial Sequence
<220>
   <221> source
   <222> 1..31
   <223> /organism="Artificial Sequence"
   /note="Primer for PCR"
   /mol_type="unassigned DNA"
<400> 65
   actctttccc tacacgacgc tcttccgatc t 31
<210> 66
   <211> 30
   <212> DNA
   <213> Artificial Sequence
<220>
   <221> source
   <222> 1..30
   <223> /organism="Artificial Sequence"
   /note="Primer for PCR"
   /mol_type="unassigned DNA"
<400> 66
   ctctttccct acacgacgct cttccgatct 30
<210> 67
   <211> 29
   <212> DNA
   <213> Artificial Sequence
<220>
   <221> source
   <222> 1..29
   <223> /organism="Artificial Sequence"
   /note="Primer for PCR"
   /mol_type="unassigned DNA"
<400> 67
   tctttcccta cacgacgctc ttccgatct 29
<210> 68
   <211> 28
   <212> DNA
   <213> Artificial Sequence
<220>
   <221> source
   <222> 1..28
   <223> /organism="Artificial Sequence"
   /note="Primer for PCR"
   /mol_type="unassigned DNA"
<400> 68
   ctttccctac acgacgctct tccgatct 28
<210> 69
   <211> 27
   <212> DNA
   <213> Artificial Sequence
<220>
   <221> source
   <222> 1..27
   <223> /organism="Artificial Sequence"
   /note="Primer for PCR"
   /mol_type="unassigned DNA"
<400> 69
   tttccctaca cgacgctctt ccgatct 27
<210> 70
   <211> 26
   <212> DNA
   <213> Artificial Sequence
<220>
   <221> source
   <222> 1..26
   <223> /organism="Artificial Sequence"
   /note="Primer for PCR"
   /mol_type="unassigned DNA"
<400> 70
   ttccctacac gacgctcttc cgatct 26
<210> 71
   <211> 25
   <212> DNA
   <213> Artificial Sequence
<220>
   <221> source
   <222> 1..25
   <223> /organism="Artificial Sequence"
   /note="Primer for PCR"
   /mol_type="unassigned DNA"
<400> 71
   tccctacacg acgctcttcc gatct 25
<210> 72
   <211> 24
   <212> DNA
   <213> Artificial Sequence
<220>
   <221> source
   <222> 1..24
   <223> /organism="Artificial Sequence"
   /note="Primer for PCR"
   /mol_type="unassigned DNA"
<400> 72
   ccctacacga cgctcttccg atct 24
<210> 73
   <211> 23
   <212> DNA
   <213> Artificial Sequence
<220>
   <221> source
   <222> 1..23
   <223> /organism="Artificial Sequence"
   /note="Primer for PCR"
   /mol_type="unassigned DNA"
<400> 73
   cctacacgac gctcttccga tct 23
<210> 74
   <211> 22
   <212> DNA
   <213> Artificial Sequence
<220>
   <221> source
   <222> 1..22
   <223> /organism="Artificial Sequence"
   /note="Primer for PCR"
   /mol_type="unassigned DNA"
<400> 74
   ctacacgacg ctcttccgat ct 22
<210> 75
   <211> 21
   <212> DNA
   <213> Artificial Sequence
<220>
   <221> source
   <222> 1..21
   <223> /organism="Artificial Sequence"
   /note="Primer for PCR"
   /mol_type="unassigned DNA"
<400> 75
   tacacgacgc tcttccgatc t 21
<210> 76
   <211> 20
   <212> DNA
   <213> Artificial Sequence
<220>
   <221> source
   <222> 1..20
   <223> /organism="Artificial Sequence"
   /note="Primer for PCR"
   /mol_type="unassigned DNA"
<400> 76
   acacgacgct cttccgatct 20
<210> 77
   <211> 19
   <212> DNA
   <213> Artificial Sequence
<220>
   <221> source
   <222> 1..19
   <223> /organism="Artificial Sequence"
   /note="Primer for PCR"
   /mol_type="unassigned DNA"
<400> 77
   cacgacgctc ttccgatct 19
<210> 78
   <211> 18
   <212> DNA
   <213> Artificial Sequence
<220>
   <221> source
   <222> 1..18
   <223> /organism="Artificial Sequence"
   /note="Primer for PCR"
   /mol_type="unassigned DNA"
<400> 78
   acgacgctct tccgatct 18
<210> 79
   <211> 17
   <212> DNA
   <213> Artificial Sequence
<220>
   <221> source
   <222> 1..17
   <223> /organism="Artificial Sequence"
   /note="Primer for PCR"
   /mol_type="unassigned DNA"
<400> 79
   cgacgctctt ccgatct 17
<210> 80
   <211> 16
   <212> DNA
   <213> Artificial Sequence
<220>
   <221> source
   <222> 1..16
   <223> /organism="Artificial Sequence"
   /note="Primer for PCR"
   /mol_type="unassigned DNA"
<400> 80
   gacgctcttc cgatct 16
<210> 81
   <211> 15
   <212> DNA
   <213> Artificial Sequence
<220>
   <221> source
   <222> 1..15
   <223> /organism="Artificial Sequence"
   /note="Primer for PCR"
   /mol_type="unassigned DNA"
<400> 81
   acgctcttcc gatct 15
<210> 82
   <211> 14
   <212> DNA
   <213> Artificial Sequence
<220>
   <221> source
   <222> 1..14
   <223> /organism="Artificial Sequence"
   /note="Primer for PCR"
   /mol_type="unassigned DNA"
<400> 82
   cgctcttccg atct 14
<210> 83
   <211> 13
   <212> DNA
   <213> Artificial Sequence
<220>
   <221> source
   <222> 1..13
   <223> /organism="Artificial Sequence"
   /note="Primer for PCR"
   /mol_type="unassigned DNA"
<400> 83
   gctcttccga tct 13
<210> 84
   <211> 12
   <212> DNA
   <213> Artificial Sequence
<220>
   <221> source
   <222> 1..12
   <223> /organism="Artificial Sequence"
   /note="Primer for PCR"
   /mol_type="unassigned DNA"
<400> 84
   ctcttccgat ct 12
<210> 85
   <211> 11
   <212> DNA
   <213> Artificial Sequence
<220>
   <221> source
   <222> 1..11
   <223> /organism="Artificial Sequence"
   /note="Primer for PCR"
   /mol_type="unassigned DNA"
<400> 85
   tcttccgatc t 11
<210> 86
   <211> 42
   <212> DNA
   <213> Artificial Sequence
<220>
   <221> source
   <222> 1..42
   <223> /organism="Artificial Sequence"
   /note="Primer for PCR"
   /mol_type="unassigned DNA"
<400> 86
   tacgagatgt gactggagtt cagacgtgtg ctcttccgat ct 42
<210> 87
   <211> 41
   <212> DNA
   <213> Artificial Sequence
<220>
   <221> source
   <222> 1..41
   <223> /organism="Artificial Sequence"
   /note="Primer for PCR"
   /mol_type="unassigned DNA"
<400> 87
   acgagatgtg actggagttc agacgtgtgc tcttccgatc t 41
<210> 88
   <211> 40
   <212> DNA
   <213> Artificial Sequence
<220>
   <221> source
   <222> 1..40
   <223> /organism="Artificial Sequence"
   /note="Primer for PCR"
   /mol_type="unassigned DNA"
<400> 88
   cgagatgtga ctggagttca gacgtgtgct cttccgatct 40
<210> 89
   <211> 39
   <212> DNA
   <213> Artificial Sequence
<220>
   <221> source
   <222> 1..39
   <223> /organism="Artificial Sequence"
   /note="Primer for PCR"
   /mol_type="unassigned DNA"
<400> 89
   gagatgtgac tggagttcag acgtgtgctc ttccgatct 39
<210> 90
   <211> 38
   <212> DNA
   <213> Artificial Sequence
<220>
   <221> source
   <222> 1..38
   <223> /organism="Artificial Sequence"
   /note="Primer for PCR"
   /mol_type="unassigned DNA"
<400> 90
   agatgtgact ggagttcaga cgtgtgctct tccgatct 38
<210> 91
   <211> 37
   <212> DNA
   <213> Artificial Sequence
<220>
   <221> source
   <222> 1..37
   <223> /organism="Artificial Sequence"
   /note="Primer for PCR"
   /mol_type="unassigned DNA"
<400> 91
   gatgtgactg gagttcagac gtgtgctctt ccgatct 37
<210> 92
   <211> 36
   <212> DNA
   <213> Artificial Sequence
<220>
   <221> source
   <222> 1..36
   <223> /organism="Artificial Sequence"
   /note="Primer for PCR"
   /mol_type="unassigned DNA"
<400> 92
   atgtgactgg agttcagacg tgtgctcttc cgatct 36
<210> 93
   <211> 35
   <212> DNA
   <213> Artificial Sequence
<220>
   <221> source
   <222> 1..35
   <223> /organism="Artificial Sequence"
   /note="Primer for PCR"
   /mol_type="unassigned DNA"
<400> 93
   tgtgactgga gttcagacgt gtgctcttcc gatct 35
<210> 94
   <211> 34
   <212> DNA
   <213> Artificial Sequence
<220>
   <221> source
   <222> 1..34
   <223> /organism="Artificial Sequence"
   /note="Primer for PCR"
   /mol_type="unassigned DNA"
<400> 94
   gtgactggag ttcagacgtg tgctcttccg atct 34
<210> 95
   <211> 33
   <212> DNA
   <213> Artificial Sequence
<220>
   <221> source
   <222> 1..33
   <223> /organism="Artificial Sequence"
   /note="Primer for PCR"
   /mol_type="unassigned DNA"
<400> 95
   tgactggagt tcagacgtgt gctcttccga tct 33
<210> 96
   <211> 32
   <212> DNA
   <213> Artificial Sequence
<220>
   <221> source
   <222> 1..32
   <223> /organism="Artificial Sequence"
   /note="Primer for PCR"
   /mol_type="unassigned DNA"
<400> 96
   gactggagtt cagacgtgtg ctcttccgat ct 32
<210> 97
   <211> 31
   <212> DNA
   <213> Artificial Sequence
<220>
   <221> source
   <222> 1..31
   <223> /organism="Artificial Sequence"
   /note="Primer for PCR"
   /mol_type="unassigned DNA"
<400> 97
   actggagttc agacgtgtgc tcttccgatc t 31
<210> 98
   <211> 30
   <212> DNA
   <213> Artificial Sequence
<220>
   <221> source
   <222> 1..30
   <223> /organism="Artificial Sequence"
   /note="Primer for PCR"
   /mol_type="unassigned DNA"
<400> 98
   ctggagttca gacgtgtgct cttccgatct 30
<210> 99
   <211> 29
   <212> DNA
   <213> Artificial Sequence
<220>
   <221> source
   <222> 1..29
   <223> /organism="Artificial Sequence"
   /note="Primer for PCR"
   /mol_type="unassigned DNA"
<400> 99
   tggagttcag acgtgtgctc ttccgatct 29
<210> 100
   <211> 28
   <212> DNA
   <213> Artificial Sequence
<220>
   <221> source
   <222> 1..28
   <223> /organism="Artificial Sequence"
   /note="Primer for PCR"
   /mol_type="unassigned DNA"
<400> 100
   ggagttcaga cgtgtgctct tccgatct 28
<210> 101
   <211> 27
   <212> DNA
   <213> Artificial Sequence
<220>
   <221> source
   <222> 1..27
   <223> /organism="Artificial Sequence"
   /note="Primer for PCR"
   /mol_type="unassigned DNA"
<400> 101
   gagttcagac gtgtgctctt ccgatct 27
<210> 102
   <211> 26
   <212> DNA
   <213> Artificial Sequence
<220>
   <221> source
   <222> 1..26
   <223> /organism="Artificial Sequence"
   /note="Primer for PCR"
   /mol_type="unassigned DNA"
<400> 102
   agttcagacg tgtgctcttc cgatct 26
<210> 103
   <211> 25
   <212> DNA
   <213> Artificial Sequence
<220>
   <221> source
   <222> 1..25
   <223> /organism="Artificial Sequence"
   /note="Primer for PCR"
   /mol_type="unassigned DNA"
<400> 103
   gttcagacgt gtgctcttcc gatct 25
<210> 104
   <211> 24
   <212> DNA
   <213> Artificial Sequence
<220>
   <221> source
   <222> 1..24
   <223> /organism="Artificial Sequence"
   /note="Primer for PCR"
   /mol_type="unassigned DNA"
<400> 104
   ttcagacgtg tgctcttccg atct 24
<210> 105
   <211> 23
   <212> DNA
   <213> Artificial Sequence
<220>
   <221> source
   <222> 1..23
   <223> /organism="Artificial Sequence"
   /note="Primer for PCR"
   /mol_type="unassigned DNA"
<400> 105
   tcagacgtgt gctcttccga tct 23
<210> 106
   <211> 22
   <212> DNA
   <213> Artificial Sequence
<220>
   <221> source
   <222> 1..22
   <223> /organism="Artificial Sequence"
   /note="Primer for PCR"
   /mol_type="unassigned DNA"
<400> 106
   cagacgtgtg ctcttccgat ct 22
<210> 107
   <211> 21
   <212> DNA
   <213> Artificial Sequence
<220>
   <221> source
   <222> 1..21
   <223> /organism="Artificial Sequence"
   /note="Primer for PCR"
   /mol_type="unassigned DNA"
<400> 107
   agacgtgtgc tcttccgatc t 21
<210> 108
   <211> 20
   <212> DNA
   <213> Artificial Sequence
<220>
   <221> source
   <222> 1..20
   <223> /organism="Artificial Sequence"
   /note="Primer for PCR"
   /mol_type="unassigned DNA"
<400> 108
   gacgtgtgct cttccgatct 20
<210> 109
   <211> 19
   <212> DNA
   <213> Artificial Sequence
<220>
   <221> source
   <222> 1..19
   <223> /organism="Artificial Sequence"
   /note="Primer for PCR"
   /mol_type="unassigned DNA"
<400> 109
   acgtgtgctc ttccgatct 19
<210> 110
   <211> 18
   <212> DNA
   <213> Artificial Sequence
<220>
   <221> source
   <222> 1..18
   <223> /organism="Artificial Sequence"
   /note="Primer for PCR"
   /mol_type="unassigned DNA"
<400> 110
   cgtgtgctct tccgatct 18
<210> 111
   <211> 17
   <212> DNA
   <213> Artificial Sequence
<220>
   <221> source
   <222> 1..17
   <223> /organism="Artificial Sequence"
   /note="Primer for PCR"
   /mol_type="unassigned DNA"
<400> 111
   gtgtgctctt ccgatct 17
<210> 112
   <211> 16
   <212> DNA
   <213> Artificial Sequence
<220>
   <221> source
   <222> 1..16
   <223> /organism="Artificial Sequence"
   /note="Primer for PCR"
   /mol_type="unassigned DNA"
<400> 112
   tgtgctcttc cgatct 16
<210> 113
   <211> 15
   <212> DNA
   <213> Artificial Sequence
<220>
   <221> source
   <222> 1..15
   <223> /organism="Artificial Sequence"
   /note="Primer for PCR"
   /mol_type="unassigned DNA"
<400> 113
   gtgctcttcc gatct 15
<210> 114
   <211> 14
   <212> DNA
   <213> Artificial Sequence
<220>
   <221> source
   <222> 1..14
   <223> /organism="Artificial Sequence"
   /note="Primer for PCR"
   /mol_type="unassigned DNA"
<400> 114
   tgctcttccg atct 14
<210> 115
   <211> 13
   <212> DNA
   <213> Artificial Sequence
<220>
   <221> source
   <222> 1..13
   <223> /organism="Artificial Sequence"
   /note="Primer for PCR"
   /mol_type="unassigned DNA"
<400> 115
   gctcttccga tct 13
<210> 116
   <211> 12
   <212> DNA
   <213> Artificial Sequence
<220>
   <221> source
   <222> 1..12
   <223> /organism="Artificial Sequence"
   /note="Primer for PCR"
   /mol_type="unassigned DNA"
<400> 116
   ctcttccgat ct 12
<210> 117
   <211> 11
   <212> DNA
   <213> Artificial Sequence
<220>
   <221> source
   <222> 1..11
   <223> /organism="Artificial Sequence"
   /note="Primer for PCR"
   /mol_type="unassigned DNA"
<400> 117
   tcttccgatc t 11
<210> 118
   <211> 58
   <212> DNA
   <213> Artificial Sequence
<220>
   <221> source
   <222> 1..58
   <223> /organism="Artificial Sequence"
   /note="Primer for PCR"
   /mol_type="unassigned DNA"
<400> 118
   aatgatacgg cgaccaccga gatctacact ctttccctac acgacgctct tccgatct 58
<210> 119
   <211> 57
   <212> DNA
   <213> Artificial Sequence
<220>
   <221> source
   <222> 1..57
   <223> /organism="Artificial Sequence"
   /note="Primer for PCR"
   /mol_type="unassigned DNA"
<400> 119
   aatgatacgg cgaccaccga gatctacact ctttccctac acgacgctct tccgatc 57
<210> 120
   <211> 56
   <212> DNA
   <213> Artificial Sequence
<220>
   <221> source
   <222> 1..56
   <223> /organism="Artificial Sequence"
   /note="Primer for PCR"
   /mol_type="unassigned DNA"
<400> 120
   aatgatacgg cgaccaccga gatctacact ctttccctac acgacgctct tccgat 56
<210> 121
   <211> 55
   <212> DNA
   <213> Artificial Sequence
<220>
   <221> source
   <222> 1..55
   <223> /organism="Artificial Sequence"
   /note="Primer for PCR"
   /mol_type="unassigned DNA"
<400> 121
   aatgatacgg cgaccaccga gatctacact ctttccctac acgacgctct tccga 55
<210> 122
   <211> 54
   <212> DNA
   <213> Artificial Sequence
<220>
   <221> source
   <222> 1..54
   <223> /organism="Artificial Sequence"
   /note="Primer for PCR"
   /mol_type="unassigned DNA"
<400> 122
   aatgatacgg cgaccaccga gatctacact ctttccctac acgacgctct tccg 54
<210> 123
   <211> 53
   <212> DNA
   <213> Artificial Sequence
<220>
   <221> source
   <222> 1..53
   <223> /organism="Artificial Sequence"
   /note="Primer for PCR"
   /mol_type="unassigned DNA"
<400> 123
   aatgatacgg cgaccaccga gatctacact ctttccctac acgacgctct tcc 53
<210> 124
   <211> 52
   <212> DNA
   <213> Artificial Sequence
<220>
   <221> source
   <222> 1..52
   <223> /organism="Artificial Sequence"
   /note="Primer for PCR"
   /mol_type="unassigned DNA"
<400> 124
   aatgatacgg cgaccaccga gatctacact ctttccctac acgacgctct tc 52
<210> 125
   <211> 51
   <212> DNA
   <213> Artificial Sequence
<220>
   <221> source
   <222> 1..51
   <223> /organism="Artificial Sequence"
   /note="Primer for PCR"
   /mol_type="unassigned DNA"
<400> 125
   aatgatacgg cgaccaccga gatctacact ctttccctac acgacgctct t 51
<210> 126
   <211> 50
   <212> DNA
   <213> Artificial Sequence
<220>
   <221> source
   <222> 1..50
   <223> /organism="Artificial Sequence"
   /note="Primer for PCR"
   /mol_type="unassigned DNA"
<400> 126
   aatgatacgg cgaccaccga gatctacact ctttccctac acgacgctct 50
<210> 127
   <211> 49
   <212> DNA
   <213> Artificial Sequence
<220>
   <221> source
   <222> 1..49
   <223> /organism="Artificial Sequence"
   /note="Primer for PCR"
   /mol_type="unassigned DNA"
<400> 127
   aatgatacgg cgaccaccga gatctacact ctttccctac acgacgctc 49
<210> 128
   <211> 48
   <212> DNA
   <213> Artificial Sequence
<220>
   <221> source
   <222> 1..48
   <223> /organism="Artificial Sequence"
   /note="Primer for PCR"
   /mol_type="unassigned DNA"
<400> 128
   aatgatacgg cgaccaccga gatctacact ctttccctac acgacgct 48
<210> 129
   <211> 47
   <212> DNA
   <213> Artificial Sequence
<220>
   <221> source
   <222> 1..47
   <223> /organism="Artificial Sequence"
   /note="Primer for PCR"
   /mol_type="unassigned DNA"
<400> 129
   aatgatacgg cgaccaccga gatctacact ctttccctac acgacgc 47
<210> 130
   <211> 46
   <212> DNA
   <213> Artificial Sequence
<220>
   <221> source
   <222> 1..46
   <223> /organism="Artificial Sequence"
   /note="Primer for PCR"
   /mol_type="unassigned DNA"
<400> 130
   aatgatacgg cgaccaccga gatctacact ctttccctac acgacg 46
<210> 131
   <211> 45
   <212> DNA
   <213> Artificial Sequence
<220>
   <221> source
   <222> 1..45
   <223> /organism="Artificial Sequence"
   /note="Primer for PCR"
   /mol_type="unassigned DNA"
<400> 131
   aatgatacgg cgaccaccga gatctacact ctttccctac acgac 45
<210> 132
   <211> 44
   <212> DNA
   <213> Artificial Sequence
<220>
   <221> source
   <222> 1..44
   <223> /organism="Artificial Sequence"
   /note="Primer for PCR"
   /mol_type="unassigned DNA"
<400> 132
   aatgatacgg cgaccaccga gatctacact ctttccctac acga 44
<210> 133
   <211> 43
   <212> DNA
   <213> Artificial Sequence
<220>
   <221> source
   <222> 1..43
   <223> /organism="Artificial Sequence"
   /note="Primer for PCR"
   /mol_type="unassigned DNA"
<400> 133
   aatgatacgg cgaccaccga gatctacact ctttccctac acg 43
<210> 134
   <211> 42
   <212> DNA
   <213> Artificial Sequence
<220>
   <221> source
   <222> 1..42
   <223> /organism="Artificial Sequence"
   /note="Primer for PCR"
   /mol_type="unassigned DNA"
<400> 134
   aatgatacgg cgaccaccga gatctacact ctttccctac ac 42
<210> 135
   <211> 41
   <212> DNA
   <213> Artificial Sequence
<220>
   <221> source
   <222> 1..41
   <223> /organism="Artificial Sequence"
   /note="Primer for PCR"
   /mol_type="unassigned DNA"
<400> 135
   aatgatacgg cgaccaccga gatctacact ctttccctac a 41
<210> 136
   <211> 40
   <212> DNA
   <213> Artificial Sequence
<220>
   <221> source
   <222> 1..40
   <223> /organism="Artificial Sequence"
   /note="Primer for PCR"
   /mol_type="unassigned DNA"
<400> 136
   aatgatacgg cgaccaccga gatctacact ctttccctac 40
<210> 137
   <211> 39
   <212> DNA
   <213> Artificial Sequence
<220>
   <221> source
   <222> 1..39
   <223> /organism="Artificial Sequence"
   /note="Primer for PCR"
   /mol_type="unassigned DNA"
<400> 137
   aatgatacgg cgaccaccga gatctacact ctttcccta 39
<210> 138
   <211> 38
   <212> DNA
   <213> Artificial Sequence
<220>
   <221> source
   <222> 1..38
   <223> /organism="Artificial Sequence"
   /note="Primer for PCR"
   /mol_type="unassigned DNA"
<400> 138
   aatgatacgg cgaccaccga gatctacact ctttccct 38
<210> 139
   <211> 37
   <212> DNA
   <213> Artificial Sequence
<220>
   <221> source
   <222> 1..37
   <223> /organism="Artificial Sequence"
   /note="Primer for PCR"
   /mol_type="unassigned DNA"
<400> 139
   aatgatacgg cgaccaccga gatctacact ctttccc 37
<210> 140
   <211> 36
   <212> DNA
   <213> Artificial Sequence
<220>
   <221> source
   <222> 1..36
   <223> /organism="Artificial Sequence"
   /note="Primer for PCR"
   /mol_type="unassigned DNA"
<400> 140
   aatgatacgg cgaccaccga gatctacact ctttcc 36
<210> 141
   <211> 35
   <212> DNA
   <213> Artificial Sequence
<220>
   <221> source
   <222> 1..35
   <223> /organism="Artificial Sequence"
   /note="Primer for PCR"
   /mol_type="unassigned DNA"
<400> 141
   aatgatacgg cgaccaccga gatctacact ctttc 35
<210> 142
   <211> 34
   <212> DNA
   <213> Artificial Sequence
<220>
   <221> source
   <222> 1..34
   <223> /organism="Artificial Sequence"
   /note="Primer for PCR"
   /mol_type="unassigned DNA"
<400> 142
   aatgatacgg cgaccaccga gatctacact cttt 34
<210> 143
   <211> 33
   <212> DNA
   <213> Artificial Sequence
<220>
   <221> source
   <222> 1..33
   <223> /organism="Artificial Sequence"
   /note="Primer for PCR"
   /mol_type="unassigned DNA"
<400> 143
   aatgatacgg cgaccaccga gatctacact ctt 33
<210> 144
   <211> 32
   <212> DNA
   <213> Artificial Sequence
<220>
   <221> source
   <222> 1..32
   <223> /organism="Artificial Sequence"
   /note="Primer for PCR"
   /mol_type="unassigned DNA"
<400> 144
   aatgatacgg cgaccaccga gatctacact ct 32
<210> 145
   <211> 31
   <212> DNA
   <213> Artificial Sequence
<220>
   <221> source
   <222> 1..31
   <223> /organism="Artificial Sequence"
   /note="Primer for PCR"
   /mol_type="unassigned DNA"
<400> 145
   aatgatacgg cgaccaccga gatctacact c 31
<210> 146
   <211> 30
   <212> DNA
   <213> Artificial Sequence
<220>
   <221> source
   <222> 1..30
   <223> /organism="Artificial Sequence"
   /note="Primer for PCR"
   /mol_type="unassigned DNA"
<400> 146
   aatgatacgg cgaccaccga gatctacact 30
<210> 147
   <211> 28
   <212> DNA
   <213> Artificial Sequence
<220>
   <221> source
   <222> 1..28
   <223> /organism="Artificial Sequence"
   /note="Primer for PCR"
   /mol_type="unassigned DNA"
<400> 147
   aatgatacgg cgaccaccga gatctaca 28
<210> 148
   <211> 27
   <212> DNA
   <213> Artificial Sequence
<220>
   <221> source
   <222> 1..27
   <223> /organism="Artificial Sequence"
   /note="Primer for PCR"
   /mol_type="unassigned DNA"
<400> 148
   aatgatacgg cgaccaccga gatctac 27
<210> 149
   <211> 26
   <212> DNA
   <213> Artificial Sequence
<220>
   <221> source
   <222> 1..26
   <223> /organism="Artificial Sequence"
   /note="Primer for PCR"
   /mol_type="unassigned DNA"
<400> 149
   aatgatacgg cgaccaccga gatcta 26
<210> 150
   <211> 58
   <212> DNA
   <213> Artificial Sequence
<220>
   <221> source
   <222> 1..58
   <223> /organism="Artificial Sequence"
   /note="Primer for PCR"
   /mol_type="unassigned DNA"
<400> 150
   caagcagaag acggcatacg agatgtgact ggagttcaga cgtgtgctct tccgatct 58
<210> 151
   <211> 57
   <212> DNA
   <213> Artificial Sequence
<220>
   <221> source
   <222> 1..57
   <223> /organism="Artificial Sequence"
   /note="Primer for PCR"
   /mol_type="unassigned DNA"
<400> 151
   caagcagaag acggcatacg agatgtgact ggagttcaga cgtgtgctct tccgatc 57
<210> 152
   <211> 56
   <212> DNA
   <213> Artificial Sequence
<220>
   <221> source
   <222> 1..56
   <223> /organism="Artificial Sequence"
   /note="Primer for PCR"
   /mol_type="unassigned DNA"
<400> 152
   caagcagaag acggcatacg agatgtgact ggagttcaga cgtgtgctct tccgat 56
<210> 153
   <211> 55
   <212> DNA
   <213> Artificial Sequence
<220>
   <221> source
   <222> 1..55
   <223> /organism="Artificial Sequence"
   /note="Primer for PCR"
   /mol_type="unassigned DNA"
<400> 153
   caagcagaag acggcatacg agatgtgact ggagttcaga cgtgtgctct tccga 55
<210> 154
   <211> 54
   <212> DNA
   <213> Artificial Sequence
<220>
   <221> source
   <222> 1..54
   <223> /organism="Artificial Sequence"
   /note="Primer for PCR"
   /mol_type="unassigned DNA"
<400> 154
   caagcagaag acggcatacg agatgtgact ggagttcaga cgtgtgctct tccg 54
<210> 155
   <211> 53
   <212> DNA
   <213> Artificial Sequence
<220>
   <221> source
   <222> 1..53
   <223> /organism="Artificial Sequence"
   /note="Primer for PCR"
   /mol_type="unassigned DNA"
<400> 155
   caagcagaag acggcatacg agatgtgact ggagttcaga cgtgtgctct tcc 53
<210> 156
   <211> 52
   <212> DNA
   <213> Artificial Sequence
<220>
   <221> source
   <222> 1..52
   <223> /organism="Artificial Sequence"
   /note="Primer for PCR"
   /mol_type="unassigned DNA"
<400> 156
   caagcagaag acggcatacg agatgtgact ggagttcaga cgtgtgctct tc 52
<210> 157
   <211> 51
   <212> DNA
   <213> Artificial Sequence
<220>
   <221> source
   <222> 1..51
   <223> /organism="Artificial Sequence"
   /note="Primer for PCR"
   /mol_type="unassigned DNA"
<400> 157
   caagcagaag acggcatacg agatgtgact ggagttcaga cgtgtgctct t 51
<210> 158
   <211> 50
   <212> DNA
   <213> Artificial Sequence
<220>
   <221> source
   <222> 1..50
   <223> /organism="Artificial Sequence"
   /note="Primer for PCR"
   /mol_type="unassigned DNA"
<400> 158
   caagcagaag acggcatacg agatgtgact ggagttcaga cgtgtgctct 50
<210> 159
   <211> 49
   <212> DNA
   <213> Artificial Sequence
<220>
   <221> source
   <222> 1..49
   <223> /organism="Artificial Sequence"
   /note="Primer for PCR"
   /mol_type="unassigned DNA"
<400> 159
   caagcagaag acggcatacg agatgtgact ggagttcaga cgtgtgctc 49
<210> 160
   <211> 48
   <212> DNA
   <213> Artificial Sequence
<220>
   <221> source
   <222> 1..48
   <223> /organism="Artificial Sequence"
   /note="Primer for PCR"
   /mol_type="unassigned DNA"
<400> 160
   caagcagaag acggcatacg agatgtgact ggagttcaga cgtgtgct 48
<210> 161
   <211> 47
   <212> DNA
   <213> Artificial Sequence
<220>
   <221> source
   <222> 1..47
   <223> /organism="Artificial Sequence"
   /note="Primer for PCR"
   /mol_type="unassigned DNA"
<400> 161
   caagcagaag acggcatacg agatgtgact ggagttcaga cgtgtgc 47
<210> 162
   <211> 46
   <212> DNA
   <213> Artificial Sequence
<220>
   <221> source
   <222> 1..46
   <223> /organism="Artificial Sequence"
   /note="Primer for PCR"
   /mol_type="unassigned DNA"
<400> 162
   caagcagaag acggcatacg agatgtgact ggagttcaga cgtgtg 46
<210> 163
   <211> 45
   <212> DNA
   <213> Artificial Sequence
<220>
   <221> source
   <222> 1..45
   <223> /organism="Artificial Sequence"
   /note="Primer for PCR"
   /mol_type="unassigned DNA"
<400> 163
   caagcagaag acggcatacg agatgtgact ggagttcaga cgtgt 45
<210> 164
   <211> 44
   <212> DNA
   <213> Artificial Sequence
<220>
   <221> source
   <222> 1..44
   <223> /organism="Artificial Sequence"
   /note="Primer for PCR"
   /mol_type="unassigned DNA"
<400> 164
   caagcagaag acggcatacg agatgtgact ggagttcaga cgtg 44
<210> 165
   <211> 43
   <212> DNA
   <213> Artificial Sequence
<220>
   <221> source
   <222> 1..43
   <223> /organism="Artificial Sequence"
   /note="Primer for PCR"
   /mol_type="unassigned DNA"
<400> 165
   caagcagaag acggcatacg agatgtgact ggagttcaga cgt 43
<210> 166
   <211> 42
   <212> DNA
   <213> Artificial Sequence
<220>
   <221> source
   <222> 1..42
   <223> /organism="Artificial Sequence"
   /note="Primer for PCR"
   /mol_type="unassigned DNA"
<400> 166
   caagcagaag acggcatacg agatgtgact ggagttcaga cg 42
<210> 167
   <211> 41
   <212> DNA
   <213> Artificial Sequence
<220>
   <221> source
   <222> 1..41
   <223> /organism="Artificial Sequence"
   /note="Primer for PCR"
   /mol_type="unassigned DNA"
<400> 167
   caagcagaag acggcatacg agatgtgact ggagttcaga c 41
<210> 168
   <211> 40
   <212> DNA
   <213> Artificial Sequence
<220>
   <221> source
   <222> 1..40
   <223> /organism="Artificial Sequence"
   /note="Primer for PCR"
   /mol_type="unassigned DNA"
<400> 168
   caagcagaag acggcatacg agatgtgact ggagttcaga 40
<210> 169
   <211> 39
   <212> DNA
   <213> Artificial Sequence
<220>
   <221> source
   <222> 1..39
   <223> /organism="Artificial Sequence"
   /note="Primer for PCR"
   /mol_type="unassigned DNA"
<400> 169
   caagcagaag acggcatacg agatgtgact ggagttcag 39
<210> 170
   <211> 38
   <212> DNA
   <213> Artificial Sequence
<220>
   <221> source
   <222> 1..38
   <223> /organism="Artificial Sequence"
   /note="Primer for PCR"
   /mol_type="unassigned DNA"
<400> 170
   caagcagaag acggcatacg agatgtgact ggagttca 38
<210> 171
   <211> 37
   <212> DNA
   <213> Artificial Sequence
<220>
   <221> source
   <222> 1..37
   <223> /organism="Artificial Sequence"
   /note="Primer for PCR"
   /mol_type="unassigned DNA"
<400> 171
   caagcagaag acggcatacg agatgtgact ggagttc 37
<210> 172
   <211> 36
   <212> DNA
   <213> Artificial Sequence
<220>
   <221> source
   <222> 1..36
   <223> /organism="Artificial Sequence"
   /note="Primer for PCR"
   /mol_type="unassigned DNA"
<400> 172
   caagcagaag acggcatacg agatgtgact ggagtt 36
<210> 173
   <211> 35
   <212> DNA
   <213> Artificial Sequence
<220>
   <221> source
   <222> 1..35
   <223> /organism="Artificial Sequence"
   /note="Primer for PCR"
   /mol_type="unassigned DNA"
<400> 173
   caagcagaag acggcatacg agatgtgact ggagt 35
<210> 174
   <211> 34
   <212> DNA
   <213> Artificial Sequence
<220>
   <221> source
   <222> 1..34
   <223> /organism="Artificial Sequence"
   /note="Primer for PCR"
   /mol_type="unassigned DNA"
<400> 174
   caagcagaag acggcatacg agatgtgact ggag 34
<210> 175
   <211> 33
   <212> DNA
   <213> Artificial Sequence
<220>
   <221> source
   <222> 1..33
   <223> /organism="Artificial Sequence"
   /note="Primer for PCR"
   /mol_type="unassigned DNA"
<400> 175
   caagcagaag acggcatacg agatgtgact gga 33
<210> 176
   <211> 32
   <212> DNA
   <213> Artificial Sequence
<220>
   <221> source
   <222> 1..32
   <223> /organism="Artificial Sequence"
   /note="Primer for PCR"
   /mol_type="unassigned DNA"
<400> 176
   caagcagaag acggcatacg agatgtgact gg 32
<210> 177
   <211> 31
   <212> DNA
   <213> Artificial Sequence
<220>
   <221> source
   <222> 1..31
   <223> /organism="Artificial Sequence"
   /note="Primer for PCR"
   /mol_type="unassigned DNA"
<400> 177
   caagcagaag acggcatacg agatgtgact g 31
<210> 178
   <211> 30
   <212> DNA
   <213> Artificial Sequence
<220>
   <221> source
   <222> 1..30
   <223> /organism="Artificial Sequence"
   /note="Primer for PCR"
   /mol_type="unassigned DNA"
<400> 178
   caagcagaag acggcatacg agatgtgact 30
<210> 179
   <211> 29
   <212> DNA
   <213> Artificial Sequence
<220>
   <221> source
   <222> 1..29
   <223> /organism="Artificial Sequence"
   /note="Primer for PCR"
   /mol_type="unassigned DNA"
<400> 179
   caagcagaag acggcatacg agatgtgac 29
<210> 180
   <211> 28
   <212> DNA
   <213> Artificial Sequence
<220>
   <221> source
   <222> 1..28
   <223> /organism="Artificial Sequence"
   /note="Primer for PCR"
   /mol_type="unassigned DNA"
<400> 180
   caagcagaag acggcatacg agatgtga 28
<210> 181
   <211> 27
   <212> DNA
   <213> Artificial Sequence
<220>
   <221> source
   <222> 1..27
   <223> /organism="Artificial Sequence"
   /note="Primer for PCR"
   /mol_type="unassigned DNA"
<400> 181
   caagcagaag acggcatacg agatgtg 27
<210> 182
   <211> 26
   <212> DNA
   <213> Artificial Sequence
<220>
   <221> source
   <222> 1..26
   <223> /organism="Artificial Sequence"
   /note="Primer for PCR"
   /mol_type="unassigned DNA"
<400> 182
   caagcagaag acggcatacg agatgt 26
<210> 183
   <211> 25
   <212> DNA
   <213> Artificial Sequence
<220>
   <221> source
   <222> 1..25
   <223> /organism="Artificial Sequence"
   /note="Primer for PCR"
   /mol_type="unassigned DNA"
<400> 183
   ttattccttc acctacacac cctgc 25
<210> 184
   <211> 23
   <212> DNA
   <213> Artificial Sequence
<220>
   <221> source
   <222> 1..23
   <223> /organism="Artificial Sequence"
   /note="Primer for PCR"
   /mol_type="unassigned DNA"
<400> 184
   agcactgtca gccgggtgcc tgg 23
<210> 185
   <211> 14
   <212> DNA
   <213> Artificial Sequence
<220>
   <221> source
   <222> 1..14
   <223> /organism="Artificial Sequence"
   /note="Primer for PCR"
   /mol_type="unassigned DNA"
<400> 185
   cgctcttccg atct 14
<210> 186
   <211> 14
   <212> DNA
   <213> Artificial Sequence
<220>
   <221> source
   <222> 1..14
   <223> /organism="Artificial Sequence"
   /note="Primer for PCR"
   /mol_type="unassigned DNA"
<400> 186
   tgctcttccg atct 14
<210> 187
   <211> 58
   <212> DNA
   <213> Artificial Sequence
<220>
   <221> source
   <222> 1..58
   <223> /organism="Artificial Sequence"
   /note="Primer for PCR"
   /mol_type="unassigned DNA"
<400> 187
   aatgatacgg cgaccaccga gatctacact ctttccctac acgacgctct tccgatct 58
<210> 188
   <211> 58
   <212> DNA
   <213> Artificial Sequence
<220>
   <221> source
   <222> 1..58
   <223> /organism="Artificial Sequence"
   /note="Primer for PCR"
   /mol_type="unassigned DNA"
<400> 188
   caagcagaag acggcatacg agatgtgact ggagttcaga cgtgtgctct tccgatct 58
<210> 189
   <211> 15
   <212> DNA
   <213> Artificial Sequence
<220>
   <221> source
   <222> 1..15
   <223> /organism="Artificial Sequence"
   /note="Primer for PCR"
   /mol_type="unassigned DNA"
<400> 189
   acctacacac cctgc 15
<210> 190
   <211> 14
   <212> DNA
   <213> Artificial Sequence
<220>
   <221> source
   <222> 1..14
   <223> /organism="Artificial Sequence"
   /note="Primer for PCR"
   /mol_type="unassigned DNA"
<400> 190
   agccgggtgc ctgg 14
<210> 191
   <211> 14
   <212> DNA
   <213> Artificial Sequence
<220>
   <221> source
   <222> 1..14
   <223> /organism="Artificial Sequence"
   /note="Primer for PCR"
   /mol_type="unassigned DNA"
<400> 191
   cgctcttccg atct 14
<210> 192
   <211> 14
   <212> DNA
   <213> Artificial Sequence
<220>
   <221> source
   <222> 1..14
   <223> /organism="Artificial Sequence"
   /note="Primer for PCR"
   /mol_type="unassigned DNA"
<400> 192
   tgctcttccg atct 14